(19) Europäisches Patentamt
European Patent Office
Office européen des brevets

(11) **EP 2 771 241 B1**

(12) **EUROPEAN PATENT SPECIFICATION**

(45) Date of publication and mention of the grant of the patent:
**18.03.2020 Bulletin 2020/12**

(21) Application number: **12805468.1**

(22) Date of filing: **29.10.2012**

(51) Int Cl.:
*A61L 27/20* (2006.01)   *A61L 27/36* (2006.01)
*A61L 27/54* (2006.01)   *A61L 31/00* (2006.01)
*A61L 31/04* (2006.01)   *A61L 31/16* (2006.01)
*A61L 24/00* (2006.01)   *A61L 24/08* (2006.01)
*B65B 3/00* (2006.01)   *B65B 3/02* (2006.01)
*A61K 31/722* (2006.01)   *A61K 31/728* (2006.01)
*A61K 45/06* (2006.01)

(86) International application number:
**PCT/IB2012/055974**

(87) International publication number:
**WO 2013/061309 (02.05.2013 Gazette 2013/18)**

(54) **NEW A-PRP MEDICAL DEVICE&TISSUE ENGINEERING COMPOSITION, MANUFACTURING MACHINES AND PROCESS**

NEUE MEDIZINISCHE A-PRP-VORRICHTUNG UND GEWEBEZÜCHTUNGSZUSAMMENSETZUNG SOWIE PRODUKTIONSMASCHINEN UND VERFAHREN DAFÜR

NOUVEAU DISPOSITIF MÉDICAL ET NOUVELLE COMPOSITION DE GÉNIE TISSULAIRE À BASE DE PRP-A, MACHINES ET PROCÉDÉ POUR LEUR FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.10.2011 GB 201118586**

(43) Date of publication of application:
**03.09.2014 Bulletin 2014/36**

(73) Proprietor: **Regen Lab SA**
**1052 Le Mont-sur-Lausanne (CH)**

(72) Inventor: **TURZI, Antoine**
**1146 Mollens (CH)**

(74) Representative: **Sach, Greg Robert**
**Valet Patent Services LLP**
**Siedlungsstrasse 4a**
**85253 Erdweg (DE)**

(56) References cited:
**EP-A1- 1 444 984     EP-A1- 2 068 268**
**EP-A1- 2 292 243     WO-A1-86/05984**
**WO-A1-2009/066102     WO-A1-2012/103100**
**WO-A2-2011/110948     FR-A1- 2 918 276**
**US-A1- 2004 103 951     US-A1- 2008 089 918**

• **Anonymous: "Ostenil plus", TRB-Chemedica , 1 December 2010 (2010-12-01), XP007922045, Retrieved from the Internet: URL:http://www.trbchemedica.com/images/stories/rheumatology/OstenilPlus/OSTENILPLUSBrochure.pdf [retrieved on 2013-07-03]**

## Description

### Field of the invention

The present invention is related to the field of tissue regeneration. It concerns more particularly new compositions or medical devices comprising or intended for the preparation of PRP, A-PRP, hyaluronic acid and/or chitosan, alone or in combination, manufacturing machines and processes thereof.

### Background of the invention

The importance of biological autologous materials in the healing process has been well documented. Most importantly, two biological autologous materials have been shown to be directly implicated in the formation of the structure of blood clots, which provide a haemostatic barrier whose role is to ensure hemostasis and seal the wound: (1) fibrin, which derives from the separation of plasma fibrinogen into two strands through the action of thrombin, and (2) the activated membranes of platelets. The wound healing process is generally presented as the succession of a coagulation phase, an inflammatory process and a regeneration process. The coagulation phase (blood clotting or clot formation) is a complex process whereby a damaged blood vessel wall is covered by a fibrin clot to stop hemorrhage and the repair of the damaged vessel is initiated by the release in large quantities of cytokines and growth factors from platelet alpha granules. The formation of blood clots (formed in physiological conditions by fibrin, platelets and red blood cells, among other blood components) is a natural phenomenon that results from tissue trauma and its role in the wound healing process, as well as in the union of bone fractures, is well-known.

Blood coagulation is the result of the complex interaction of a number of protein clotting factors through a cascade. In general, damage to the vascular endothelium exposes subendothelial structures, which attract platelets and induce them to aggregate reversibly. The protein thrombin, formed during activation of the coagulation pathway generates insoluble cross-linked fibrils of the protein fibrin and causes the platelets to aggregate irreversibly. The resulting platelet-fibrin clot is an effective barrier against loss of blood from the vascular system and also serves as a scaffold for subsequent repair of the lining of the blood vessel.

Platelet-rich plasma (PRP) can be defined as an autologous concentrate of platelets in a small volume of plasma; it has been developed as an autologous biomaterial and has proven to be useful in the healing and regeneration of tissues (Marx et al, 2004, J. Oral Maxillofac. Surg., 62, 489-496). PRP not only consists in a platelet concentrate but also contains growth factors (such as platelet-derived growth factor: PDGF, vascular endothelial growth factor: VEGF, transforming growth factor: TGF and epidermal growth factor: EGF, etc.) that are actively secreted by platelets and are known to have a fundamental role in wound healing initiation process.

Various techniques of preparation of PRP by centrifugation processes have been developed. However, due to the sensitivity of the platelet cells and the variability of the efficiency of the methods of separation of the platelets from the red blood cells, a great variability exist among the methods used for the preparation of platelet concentrates. There is also an important loss of valuable biologic tissue from the patients when PRP is prepared with old empiric or semi automatic devices. In addition, in order to obtain platelet concentrates, the use of relatively complex kits and costly dedicated machinery and the equally costly involvement of specialized technicians are required.

Therefore there is a need for the development of an hematology tube with pharmaceutical grade standards, enabling a safe collection of blood, depletion of all erythrocytes and preparation of plasma cells with high yields of platelets and leukocytes, easy to use and cost effective.

WO2011/110948 discloses the preparation of tubes comprising hyaluronic acid, enriched plasma, cell selector gel and sodium citrate for the preparation of wound healant compositions.

Further, the preparation of cells in view of cellular or tissue regeneration for use in transplantation, post-operative regeneration or for aesthetic purpose is confronted to the long-term conservation problem of cells and tissues by cryoconservation. This technique has shown serious drawbacks and problems such as crystal formation, osmotic problems, aggregation, inhibition of protein synthesis ability, stress protein expression in response to thermal stress. Therefore, tissue or cell cryoconservation is known to alter the cell viability and stability. Some of the cryoconservation side effects may be limited by the use of anti-freezing agents such as DMSO or glycerol or other cryopreservatives but the concentration of these agents has to be adapted to limit their toxicity and side effects. Then cells are washed and dispensed with PBS or other biologic preparation, the cells survival rate published in literature is low. In any case those cells cannot produce an efficient PRP, because the physician dispenses fresh and viable cells in a plasma full of nutritive protein for cell expansion.

The current manual manufacturing processes of PRP kits are fastidious, time-consuming and necessitate the intervention of various human resources. In addition, due to the exposure of many sources of contamination during the manufacturing process, the US-FDA have clearly set as standard the obligation to guarantee a bioburden control for the quality of medical devices that shall be "pyrogen free". Therefore, there was a need in the development of new machines for hematological tubes for the manufacturing of pharmaceutical grade standards and with constant, predictable and reliable biological results.

In addition, such new machines for automated manufacturing processes of PRP kits, shall be able to provide a sterility control output and increase the quality of the PRP preparations by the physicians and the medical environment.

In addition to the A-PRP (Autologous Platelet Rich Plasma) or autologous preparation of fresh cells, there is a need of mechanical and biological support to the cells migration and expansion eventually differentiation. Cells and growth factors need a provisory matrix to enhance their biological expansion.

**Summary of the invention**

The invention relates to the field of tissue regeneration. It concerns more particularly new manufacturing processes, tubes and devices for thrombin, platelet concentrate and wound healant preparations, compositions, PRP compositions, A-PRP compositions and uses thereof. The invention is defined by the appended claims.

In one aspect, the disclosure provides a filling machine for the automated preparation of hematology tubes characterized in that the filling machine comprises:

    a. an apparatus for moving empty or prefilled hematology tubes one by one,
    b. at least one injector for injecting in the hematology tubes either:

        i. an anticoagulant,
        ii. a polymer and an anticoagulant,
        iii. hyaluronic acid and/or chitosan and an anticoagulant, or
        iv. hyaluronic acid and/or chitosan, a polymer and an anticoagulant

    c. optionally at least one apparatus for controlled vacuum and clogging of the hematology tubes.

In another aspect, the disclosure provides a sealing machine for the automated double blister packaging of a medical device, PRP or autologous PRP (A-PRP) kit with accessories characterized in that the sealing machine comprises 2 parallel carpets.

In another aspect, the disclosure provides a method of automatically manufacturing hematology tubes by means of a filling machine comprising:

    a. filling the hematological tubes with either:

        i. an anticoagulant,
        ii. a polymer and an anticoagulant,
        iii. hyaluronic acid and/or chitosan and an anticoagulant, or
        iv. hyaluronic acid and/or chitosan, a polymer and an anticoagulant,

    b. optionally allowing controlled vacuum and clogging of the hematology tubes.

In another aspect, the disclosure provides a method of automatically manufacturing a PRP or A-PRP kit or medical device by means of a sealing machine for double blister packaging of hematological tubes with accessories comprising:

    a. molding, filling, sealing and cutting of a first blister comprising the hematological tubes with accessories,
    b. molding, sealing and cutting of a second blister on the first blister.

In another aspect, the disclosure provides a method of automatically manufacturing a PRP or A-PRP kit or medical device, comprising:

    a. moving empty or prefilled hematology tubes one by one,
    b. filling the hematological tubes with either:

        i. an anticoagulant,
        ii. a polymer and an anticoagulant,
        iii. hyaluronic acid and/or chitosan and an anticoagulant, or
        iv. hyaluronic acid and/or chitosan, a polymer and an anticoagulant,

c. optionally allowing controlled vacuum and clogging of the hematology tubes,

d. molding, filling, sealing and cutting of a first blister comprising the hematological tubes with accessories, and

e. molding, sealing and cutting of a second blister on the first blister.

In another aspect, the disclosure provides a composition comprising hyaluronic acid of about 1400 KDa to about 1600 KDa at about 1.8% to about 2.2% concentration and optionally a platelet concentrate, preferably platelet rich plasma.

In another aspect, the disclosure provides a composition comprising hyaluronic acid of about 4000 KDa or above 4000 KDa at about 1.8% to about 2.2% concentration, more preferably of about 4000 KDa at about 2% concentration.

The invention provides a composition comprising at least one low molecular weight hyaluronic acid and at least one high molecular weight hyaluronic acid characterized in that:

- said low molecular weight hyaluronic acid is less than 600 KDa or about 600 KDa, and
- said high molecular weight hyaluronic acid is about 4000 Kda or above 4000 KDa (about at least 4000 kDa), wherein the ratio of low molecular weight hyaluronic acid to high molecular weight hyaluronic acid is 2:3 and wherein the total concentration is 2.2% to 2.8%, and further comprising: i) an anticoagulant and/or a thixotropic gel.

In another aspect, the disclosure provides a wound healant composition, tissue healant composition, cell composition, composition, PRP composition, A-PRP composition, thrombin serum, or haemostatic agent obtained by using a hematology tube, PRP kit, A-PRP kit or medical device according to any of the previous aspects, obtained by using a machine according to any of the previous aspects or obtained by a method according to any of the previous aspects for use in dentistry, orthopedics, sports medicine, cosmetics, esthetics, surgery, ophthalmology and/or mesotherapy.

## Description of the figures

The accompanying drawings, which are incorporated herein and form a part of the specification illustrate preferred embodiments of the present invention, and together with the description, serve to explain the principles of the invention.

Figure 1. Schematic view of the filling machine. Figure 1-A represents a peristaltic pump (piston pump) with a first injector allowing injection of a polymer in the hematology tube. Figure 1-B represents a pressurized pre-filling tank for the gel or polymer to be injected. Figure 1-C represents the second injector allowing injection of an anticoagulant (e.g. citrate) in the hematology tubes. Figure 1-D represents a vibration cup or 2 bell-type shaped components (Puck & Place station with vibrator) allowing a controlled vacuum and the clogging of the tubes.

Figure 2. Detailed view of the filling machine. The filling machine comprises empty pucks (Figure 2-1), a counterclock wise (CCW) inlet rotary table (Figure 2-2), location where the pucks with tubes are inserted and enter a CCW rotating wheel (Figure 2-3), a hot dose station (Figure 2-4), a hot hose (Figure 2-5), a pressurized tank (Figure 2-6), a peristaltic pump (Figure 2-7), a second dosing station (Figure 2-8), a Puck & Place station (Figure 2-9), a vibrator (Figure 2-10).

Figure 3. Detailed view of the sealing machine. The sealing machine comprises a bottom web film (Figure 3-1), a preheating and forming station (Figure 3-2), a sealing station (Figure 3-3), a camerasystem (Figure 3-4), a top web film (Figure 3-5), a printer (Figure 3-6), an operating board (Figure 3-7), a die cut station (Figure 3-8), a scrap web winder (Figure 3-9), a discharge conveyor belt (Figure 3-10), and a picker (Figure 3-11).

Figure 4. Volume in ml of blood withdrawn and of PRP or PRP-HA MIX obtained for BCT alone and the three subsets BCT-HA A, BCT-HA B and BCT-HA C.

Figure 5. pH of mean blood and PRP at time T=0 and time T=4 hours for whole blood, Regen BCT PRP and Regen BCT-HA MIX.

Figure 6. RBC concentration in blood and PRP ($10^6/mm^3$) at time T=0 and time T=4 hours for whole blood, Regen BCT PRP, Regen BCT-HA PRP and Regen BCT-HA MIX.

Figure 7. White Blood Cells (WBC) concentration in blood and PRP ($10^3/mm^3$) at time T=0 and time T=4 hours for whole blood, Regen BCT PRP, Regen BCT-HA PRP and Regen BCT-HA MIX.

Figure 8. White Blood Cells (WBC) recovery in % at time T=0 and time T=4 hours for Regen BCT PRP and Regen BCT-HA PRP.

Figure 9. White Blood Cells (WBC) repartition for the subsets granulocytes (GRA) and monocytes (MNC) in blood and PRP at time T=0 and time T=4 hours for whole blood, Regen BCT PRP and Regen BCT-HA PRP.

Figure 10. White Blood Cells (WBC) subsets recovery (granulocytes (GRA) and monocytes (MNC)) in % at time T=0 for Regen BCT PRP and Regen BCT-HA PRP.

Figure 11. Platelet (PLT) concentration in blood and PRP($10^3/mm^3$) at time T=0 and time T=4 hours for whole blood, Regen BCT PRP, Regen BCT-HA PRP and Regen BCT-HA MIX.

Figure 12. Platelet concentration factor at time T=0 and time T=4 hours for Regen BCT PRP, Regen BCT-HA PRP and Regen BCT-HA MIX.

Figure 13. Platelet recovery in % at time T=0 and time T=4 hours for Regen BCT PRP and Regen BCT-HA PRP.

Figure 14. CD62P expression on platelets with or without ADP at time T=0 and time T=4 hours for Regen BCT PRP and Regen BCT-HA MIX.

Figure 15. Hypotonic stress response (difference in absorbance at 405 nm) at time T=0 and time T=4 hours for Regen BCT PRP and Regen BCT-HA MIX.

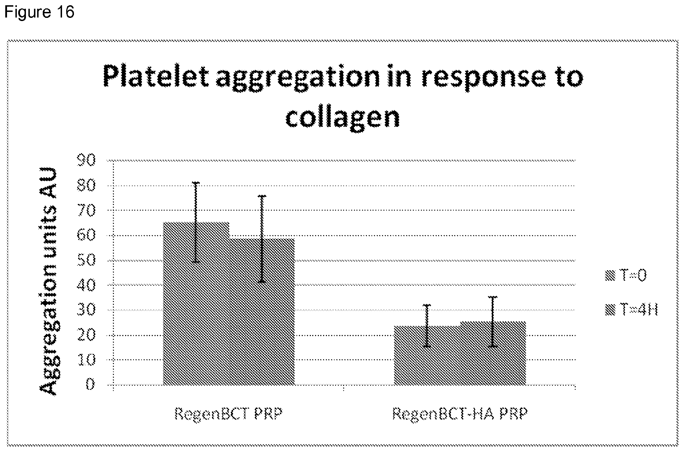

Figure 16. Platelet aggregation in response to collagen (aggregation units AU) at time T=0 and time T=4 hours for Regen BCT PRP and Regen BCT-HA PRP.

Figure 17. Viscosity in mPa.S for various hyaluronic acids (HA1 to HA4) differing in molecular weight and for various concentrations (2%, 3% or 4%).

Figure 18. Viscosity of various hyaluronic acids (HA1 to HA4, 500 KDa and 4000 KDa) plotted against concentration (1% to 4%).

Figure 19. Release in pg/ml of respectively PDGF-AA, EGF and PDGF-BB at day 0 (T0) for a PRP preparation alone (BCT T0) versus a combination of PRP and HA (BCT+IALUR T0).

Figure 20. Release in pg/ml of PDGF-BB for two cases at day 0 (T0), day 3 (T3) and day 5 (T5) for a PRP preparation alone (BCT) versus a combination of PRP and HA (BCT HA).

Figure 21. Release in pg/ml of EGF for three different patients (patients 1, 2 or 3) at day 0 (T0), day 3 (T3), day 5 (T5), day 7(T7) and day 10 (T10) for a PRP preparation alone (BCT) in Figure 21-A versus a combination of PRP and HA (BCT HA) in Figure 21-B. Median release in pg/ml of EGF at day 0 (T0), day 3 (T3), day 5 (T5), day 7(T7) and day 10 (T10) for a PRP preparation alone (BCT) versus a combination of PRP and HA (BCT HA) in Figure 21-C.

**Detailed description of the invention**

The following paragraphs provide definitions of the terms according to the invention and are intended to apply uniformly throughout the specification and claims unless an otherwise expressly set out definition provides a broader definition.

The expression "thixotropic" means a gel that becomes more fluid as a result of agitation or pressure, i.e. a gel which viscosity is decreasing as a result of agitation or pressure. The term viscosity refers to those characteristics of the specified material(s) determining the degree of gelation, such as for example the firmness or hardness of the material, the degree to which the material resists flowing like a fluid. A thixotropic gel according to the invention comprising a polyester gel or a mixture thereof which is water insoluble and chemically inert to blood constituents which can be used in accordance with the invention. Typical thixotropic gels are used in blood cells separation for diagnostics and proteomics purposes. A thixotropic gel is also herein referred to as a "cell selector gel". Other gels may be used in the present disclosure.

The expression "point-of-care" means all services provided to patients at the bedside. The expression "phlebotomy accessories" or "venipuncture accessories" means accessories that allow the puncture of a vein with a needle for the purpose of drawing blood.

Alternative expressions for "wound healant" or "wound sealant" or "tissue healant" or "tissue sealant" or "wound healing composition" or "tissue healing composition" are "bioadhesive sealant" or "fibrin glue".

The expression "wound healant" or "wound sealant" or "tissue healant" or "tissue sealant" or "wound healing composition" or "tissue healing composition" or "bioadhesive sealant" or "fibrin glue" means an agent or a composition that is able to promote and/or increase the speed and/or quality of cicatrisation of a wound. Wound healants or sealants are able to promote tissue regeneration. The expression "wound" means any damaged tissue, for example following trauma or surgery. Wounds in mammals, include for examples bed sores, ulcers, lacerations and burns, graft sites (graft donor and acceptor sites), fistulas, periodontal tissue damages, diabetic non- healing wounds, consequences of traumas or any surgery act. In its general sense the expression is intended to also encompass skin damages where the skin surface presents some depression without necessarily a cut on its surface such as age-related tissue damages (e.g. wrinkles) and scars such as for example acne (especially after dermabrasion treatment) or rubella scars.

The expression "PRP" means a platelet-rich-plasma, preferably of mammal origin or human origin, more preferably autologous, prepared by the process of the invention in order to pellet and remove erythrocytes and concentrate the plasma in leucocytes, thrombocytes and adhesion proteins as compared to native whole blood. The expression "autologous" or "autogenic" or "autogenous" means an in-vivo method wherein a single donor's blood, tissue and/or cell is used and wherein the blood, tissue and/or cell extracted from this donor is intended for use on the same donor. A-PRP herein means Autologous Platelet Rich Plasma. As opposed, "allogeneic" methods are using blood, tissue and/or cell from one or more third parties for use on a donor ("homologous" or "heterologous"). An autologous product avoids some of the common problems associated with the use of biological materials from third parties, such as for example screening to assure that the donor was biologically or immunologically compatible with the patient and potential contamination with hepatitis, HIV, prion, Creutzfeld- Jacob's disease and the like. The expression "coagulation activator" means an agent, for example an enzyme, that is able to trigger or activate coagulation of plasma and platelets aggregation. A coagulation activator comprises a thrombin activator and/or a fibrinogen activator and/or thrombin and/or an autologous thrombin and/or an autologous thrombin serum and/or calcium chloride and/or calcium gluconate and/or calcium saccharate. Coagulation may be combined in order to change the stiffness of compositions.

The expression "thrombin activator" means an agent that is able to activate thrombin and to trigger coagulation. Typical thrombin activators are certain co factors such as sodium or calcium. In practicing this invention, thrombin activation preferably occurs in the presence of calcium ions. Calcium ions are generally added to the platelet concentrate as a salt solution to provide a final concentration generally of or about 0.1 mg/mL of platelet concentrate. Suitable calcium salts include, without limitation, $CaCO_3$, $CaSO_4$ or $CaCl_2$. A preferred calcium salt for use in the invention is calcium gluconate (CaGL). CaGL is available as calcium gel injection, USP 10% (Regen Lab, Switzerland). The expression "fibrinogen activator" means an agent that is able to activate the conversion of fibrinogen into fibrin and triggers the formation of the clot. Typical fibrinogen activators are thrombin or batroxobin. The term thrombin may include calcified thrombin, in particular, from or about 100 to about 10 units of thrombin per 1 mL of 10% of aqueous calcium gluconate solution; it may include calcified bovine thrombin, allogeneic thrombin or recombinant human thrombin, preferably autologous thrombin. A fibrinogen activator can be an enriched thrombin composition such as thrombin compositions as described in US 6,472,162 or an autologous thrombin serum according to the invention. The expression "therapeutically effective amount" means the amount or amounts of the constituent elements or combination thereof necessary to enhance wound healing such as, for example, the reduction in the volume or surface area of a wound, the increase in the amount of granulation tissue or other biological material facilitating collagen lay down, vascular in growth, fibroblast proliferation or overall healing; All of the versions of the invention described herein are assumed to have the therapeutically effect amount(s) of constituent substances, or combinations thereof. By the expression "pharmaceutically acceptable carrier" is intended pharmaceutically acceptable additional ingredient such as stabilizers, antimicrobial agents, buffers, adjuvants, anaesthetics, corticosteroids and the like. By the expression "cosmetically acceptable carrier" is intended cosmetically acceptable additional ingredient such as stabilizers, buffers, colouring agents, flavouring agents, adjuvants, and the like.

The expression "Cyclic Olefin Copolymer" (COC) or "Cyclic Olefin Polymer" (COP) means an amorphous polymer, Ethylene Copolymer; COC; COP; Cyclo Olefinecopolymer; Cyclic Olefin Polymer; Ethylene-norbornene Copolymer. COPs use a single type of monomer whereas COCs use different types of monomers. The invention encompasses cyclic olefin copolymers based on different types of cyclic monomers and polymerization methods. The Cyclic olefin copolymers or polymers of the present invention may be produced by chain copolymerization of cyclic monomers such as 8,9,10-trinorborn-2-ene (norbornene) or 1,2,3,4,4a,5,8,8a-octahydro-1,4:5,8-dimethanonaphthalene with ethene, Ticona's TOPAS, Mitsui Chemical's APEL, or by ring-opening metathesis polymerization of various cyclic monomers followed by hydrogenation (for example Japan Synthetic Rubber's ARTON, Zeon Chemical's Zeonex and Zeonor).

The expression "hyaluronic acid" (also called hyaluronan or hyaluronate) means an anionic, nonsulfated glycosaminoglycan distributed widely throughout connective, epithelial, and neural tissues. It is unique among glycosaminoglycans in that it is nonsulfated, forms in the plasma membrane instead of the Golgi, and can be very large, with its molecular weight often reaching the million. One of the chief components of the extracellular matrix, hyaluronan contributes significantly to cell proliferation and migration.

The expression "chitosan" means a linear polysaccharide composed of randomly distributed β-(1-4)-linked D-glucosamine (deacetylated unit) and N-acetyl-D-glucosamine (acetylated unit). Chitosan is produced commercially by deacetylation of chitin , which is the structural element in the exoskeleton of crustaceans (crabs, shrimp, etc.) and cell walls of fungi. The degree of deacetylation (%DD) can be determined by NMR spectroscopy, and the %DD in commercial chitosans is in the range 60-100 %. On average, the molecular weight of commercially produced chitosan is between 3800 to 20,000 daltons. A common method for the synthesis of chitosan is the deacetylation of chitin using sodium hydroxide in excess as a reagent and water as a solvent. This reaction pathway, when allowed to go to completion (complete deacetylation) yields up to 98% product. The amino group in chitosan has a pKa value of -6.5, which leads to a protonation in acidic to neutral solution with a charge density dependent on pH and the %DA-value. This makes chitosan water soluble and a bioadhesive which readily binds to negatively charged surfaces such as mucosal membranes. Chitosan enhances the transport of polar drugs across epithelial surfaces, and is biocompatible and biodegradable.

BCT herein stands for Blood Cell Therapy(ies) or Blood Collection Tube(s).

In one aspect, the disclosure provides a filling machine for the automated preparation of hematology tubes characterized in that the filling machine comprises:

> a. at least one injector for injecting in the hematology tubes either:

> > i. an anticoagulant,
> > ii. a polymer and an anticoagulant,
> > iii. hyaluronic acid and/or chitosan and an anticoagulant, or
> > iv. hyaluronic acid and/or chitosan, a polymer and an anticoagulant

> b. optionally at least one apparatus for controlled vacuum and clogging of the hematology tubes.

In one aspect, the disclosure provides a filling machine for the automated preparation of hematology tubes characterized in that the filling machine comprises:

> a. at least one injector for injecting in the hematology tubes either:

> > v. an anticoagulant,
> > vi. a polymer and an anticoagulant,
> > vii. hyaluronic acid and/or chitosan and an anticoagulant, or
> > viii. hyaluronic acid and/or chitosan, a polymer and an anticoagulant

> b. optionally 2 bell-type shaped components for controlled vacuum and clogging of the hematology tubes.

In one aspect, the invention provides a filling machine for the automated preparation of hematology tubes characterized in that the filling machine comprises:

> a. an apparatus for moving empty or prefilled hematology tubes one by one,
> b. at least one injector for injecting in the hematology tubes either:

> > i. an anticoagulant,
> > ii. a polymer and an anticoagulant,
> > iii. hyaluronic acid and/or chitosan and an anticoagulant, or
> > iv. hyaluronic acid and/or chitosan, a polymer and an anticoagulant

> c. optionally 2 bell-type shaped components for controlled vacuum and clogging of the hematology tubes.

In one aspect, the disclosure provides a filling machine for the automated preparation of hematology tubes characterized in that the filling machine comprises:

> a. optionally an injector for injecting hyaluronic acid and/or chitosan in the hematology tubes,
> b. optionally an injector for injecting a polymer in the hematology tubes,
> c. an injector for injecting an anticoagulant in the hematology tubes, and
> d. optionally 2 bell-type shaped components for controlled vacuum and clogging of the hematology tubes.

In one aspect, the disclosure provides a filling machine for the automated preparation of hematology tubes characterized in that the filling machine comprises only one injector for injecting an anticoagulant in the hematology tubes.

In one aspect, the disclosure provides a filling machine for the automated preparation of hematology tubes characterized in that the filling machine comprises at least one injector for injecting at least an anticoagulant in the hematology tubes. In one aspect, the invention provides a filling machine for the automated preparation of hematology tubes characterized in that the filling machine comprises:

a. a rotating wheel for moving empty or prefilled hematology tubes one by one,
b. at least one injector for injecting in the hematology tubes either:

i. an anticoagulant,
ii. a polymer and an anticoagulant,
iii. hyaluronic acid and/or chitosan and an anticoagulant, or
iv. hyaluronic acid and/or chitosan, a polymer and an anticoagulant

c. optionally 2 bell-type shaped components for controlled vacuum and clogging of the hematology tubes.

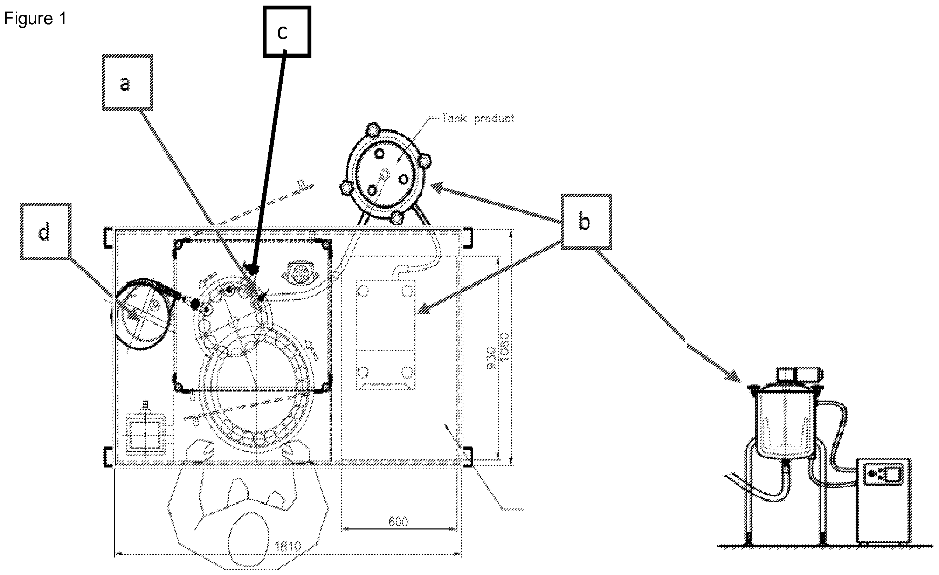

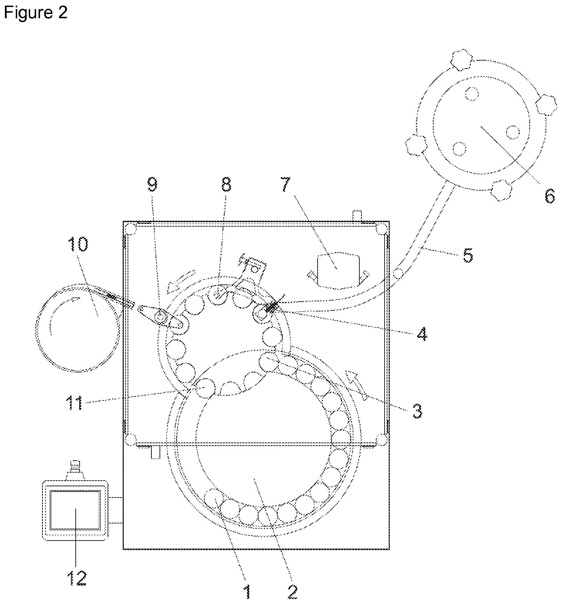

Figures 1 and 2 represent detailed views of the filling machine.

Preferably, the disclosure provides a filling machine according to the previous claims characterized in that the filling machine comprises:

a. a rotating wheel for moving the hematology tubes one by one,
b. a first injector for injecting a polymer in the hematology tubes,
c. a second injector for injecting an anticoagulant in the hematology tubes,
d. optionally 2 bell-type shaped components for controlled vacuum and clogging of the hematology tubes.

In one aspect, the manufacturing method is further characterized by an apparatus, preferably a rotating wheel, for moving empty or prefilled hematology tubes one by one.

In one aspect, the manufacturing method is further characterized by an apparatus, preferably a 2 bell-type shaped components, for controlled vacuum and/or clogging of the hematology tubes.

The hyaluronic acid and/or chitosan, polymer and/or anticoagulant may be injected sequentially or simultaneously, by one or more injectors. Preferably, the hyaluronic acid and/or chitosan, polymer and/or anticoagulant are injected sequentially.

In one aspect of the disclosure, the filling machine comprises one single injector permitting the simultaneous or sequential injection of hyaluronic acid and/or chitosan, polymer and/or anticoagulant.

Advantageously, the manufacturing process for sterile hematological tube enables the processing of 4 individual tasks simultaneously or sequentially in sterile conditions.

The filling machine used enables filling of the hematological tubes with polymer and citrate, vacuum filling and clogging in an automated single operation.

The filling machine is firstly characterized by carousels allowing movement of the tubes one by one (Figure 2-1, 2-2 and 2-3). Secondly, the machine is characterized by a first injector allowing injection of a polymer in the hematology tube (Figure 1-A and 1-B; Figure 2-4, 2-5 and 2-6). Thirdly, the machine is characterized by a second injector allowing injection of citrate in the hematology tube Figure 1-C; Figure 2-7 and 2-8). Further embodiments may include other injectors or injectors that inject equivalents or other substances. Fourthly, the machine is characterized by 2 bell-type shaped components allowing (Figure 1-D; Figure 2-10):

i) a controlled vacuum in the tubes. In one embodiment, the controlled vacuum is for tubes of 8 to 9 ml capacity.
ii) Clogging of the tubes. Advantageously, this enables conservation of vacuum. The machine is further characterized by a bowl feeder feeding the bell-type shaped components with plugs and protection caps (Figure 2-9 and 2-10).

In another aspect, the invention provides a filling machine for the automated preparation of hematology tubes characterized in that the filling machine comprises pucks (Figure 2-1), a first rotating apparatus, preferably a counterclock wise (CCW) inlet rotary table (Figure 2-2), a second rotating apparatus linked to the first rotating apparatus, preferably a CCW rotating wheel (Figure 2-3), and at least one injector for injecting substances inside the hematology tubes.

In another aspect, the invention provides a filling machine for the automated preparation of hematology tubes characterized in that the filling machine comprises pucks (Figure 2-1), a counterclock wise (CCW) inlet rotary table (Figure 2-2), a CCW rotating wheel (Figure 2-3), a hot dose station (Figure 2-4), a hot hose (Figure 2-5), a pressurized tank (Figure 2-6), a peristaltic pump (Figure 2-7), a second dosing station (Figure 2-8), a Puck & Place station (Figure 2-9) and a vibrator (Figure 2-10).

Injector may also be referred herein as a dosing station.

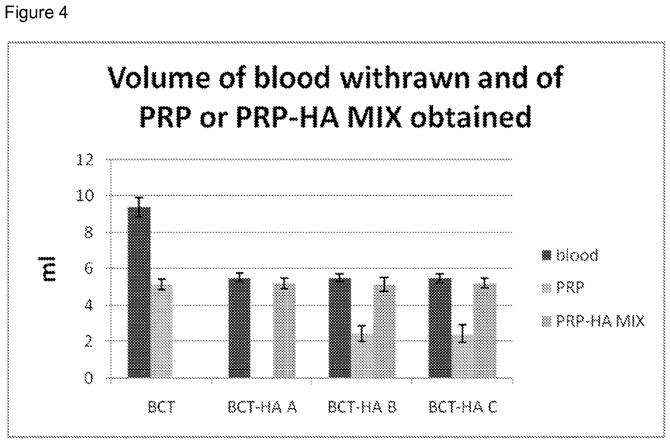

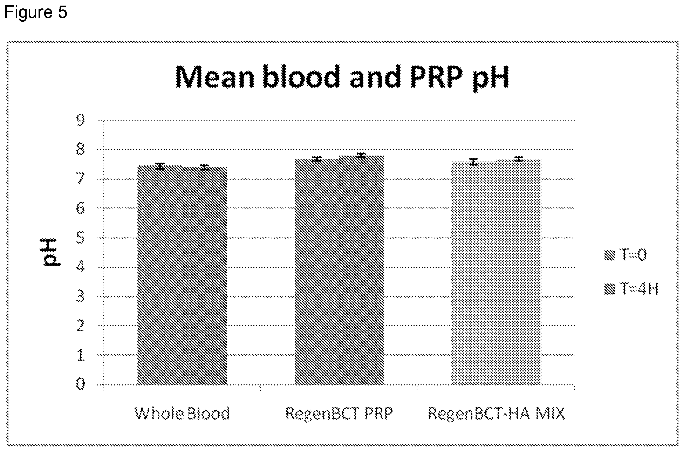

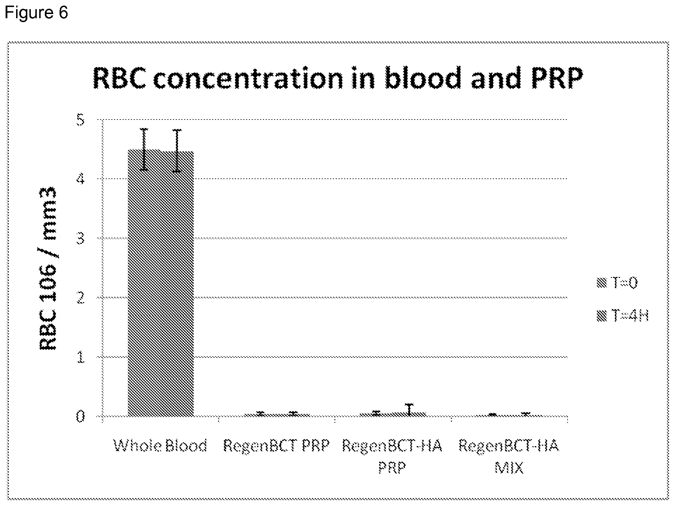

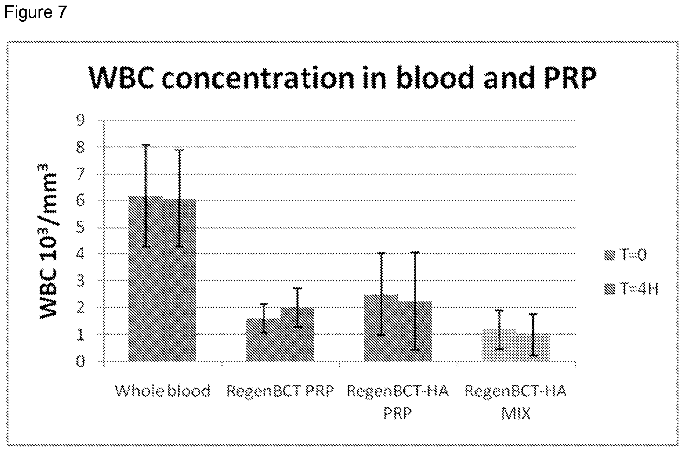

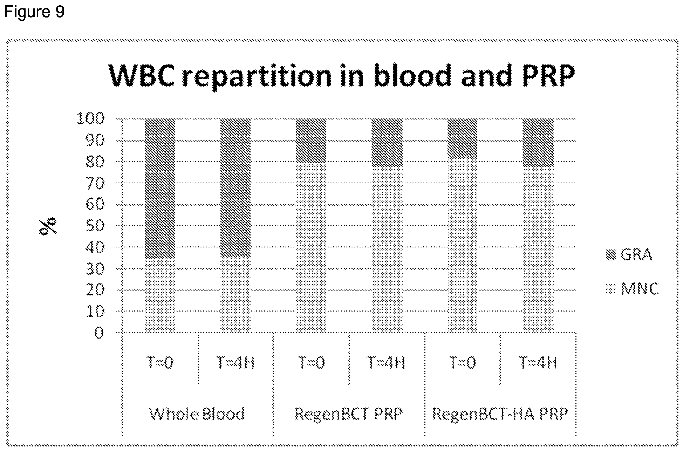

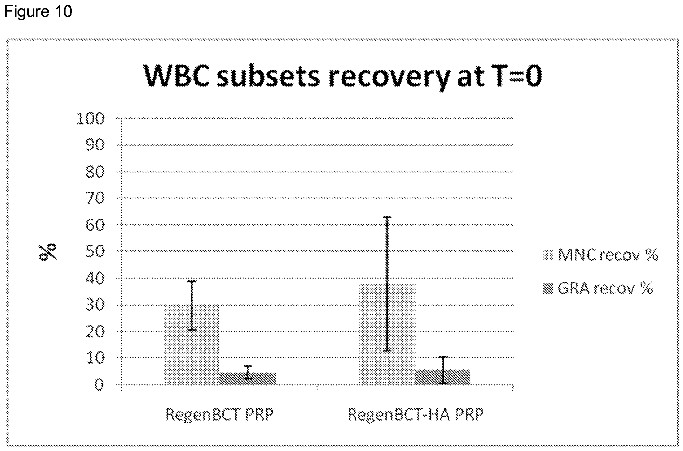

Injection of substances inside the hematology tubes may comprise one or more dose stations, one or more hot dose station (Figure 2, 4), one or more pressurized tanks (Figure 2, 6), one or more hoses, one or more hot hoses (Figure 2, 5), one or more nozzles, one or more peristaltic pumps (Figure 2, 7), one or more Pick and Place (P&P) stations (Figure 2, 9), one or more vibrators (Figure 2, 10), and/or one or more rods in the pick arm of the P&P.

Advantageously, the manufacturing process requires only a small working area, approximately 2 square meters. Advantageously, the manufacturing process is bioburden controlled (number of particles) in a clean room ISO 8. Advantageously, the manufacturing process may be performed under laminar flux and the working area consequently is advantageously in conformity with ISO 5. Further advantageously, the manufacturing process is controlled by only one single technician.

In one aspect, the filling machine is controlled through a display, preferably a HMI touchscreen display (Figure 2-12).

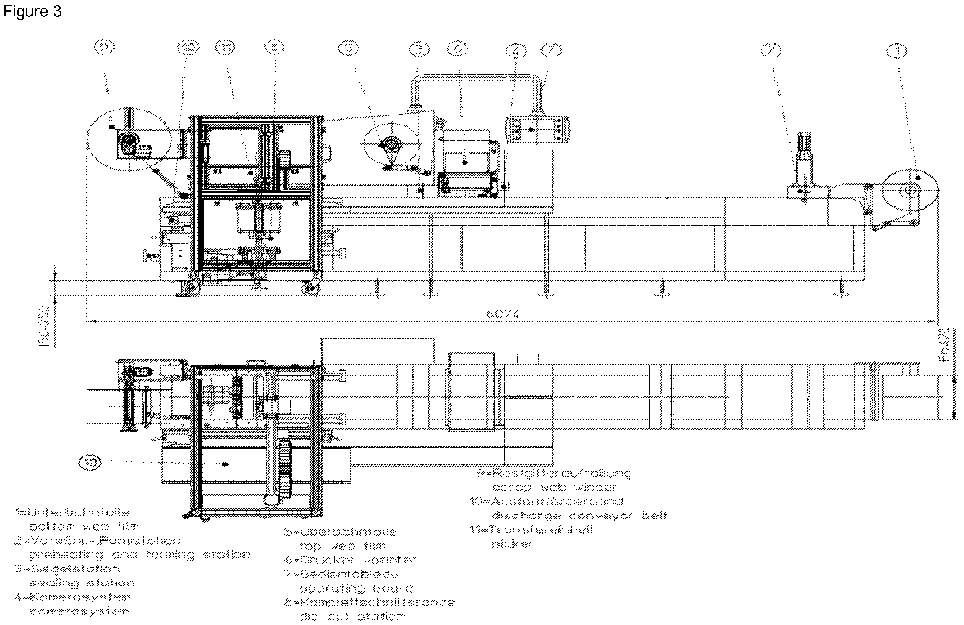

In another aspect, the disclosure provides a sealing machine for the automated double blister packaging of a medical device, PRP kit or A-PRP (Autologous Platelet Rich Plasma) kit with accessories characterized in that the sealing machine comprises 2 parallel carpets. Figure 3 represents a detailed view of the sealing machine. Kit may herein be substituted by medical device.

In another aspect, the disclosure provides a sealing machine for the automated double blister packaging of a medical device, PRP kit or A-PRP kit with accessories characterized in that the sealing machine comprises 2 parallel carpets and a Pick & Place (P&P) apparatus.

Advantageously, uniqueness is achieved in the manufacturing process of the hematology tube by the use of two machines called a filling machine and a sealing machine.

The sealing machine used enables double blister packaging in an automated single operation.

The sealing machine is characterized by 2 parallel carpets allowing the following operations (Figure 3):

    i) Formation of a first shell or blister made of APET or PETG film
    ii) Filling of the first blister with the hematological tubes coming from the filing machine with accessories.
    iii) Sealing of the first blister with a tyvek film
    iv) Cutting of the tyvek film in order to adopt the appropriate shape
    v) Formation, sealing and cutting of a second blister on the first blister.

Advantageously, the sealing machine enables the formation, filling, sealing and cutting of a blister comprising hematological tubes with accessories.

In one aspect, only one blister is used. Preferably, 2 blisters are used for conformity with ISO norms.

The sealing machine is further characterized by a pick and place transfer system (P&P apparatus) allowing transfer of the blister to its initial position on the first carpet with the use of a second carpet. This step allows the formation of a second blister by the machine which will be further sealed and ready for the printing process. The two carpets with the P&P apparatus enable the transfer of the first blister already sealed and cut and containing the hematology tubes and accessories to its initial position for further processing, i.e. for the formation, sealing and cutting of the second blister on the first blister for double blister packaging.

Advantageously, the sealing machine enables the processing of individual tasks simultaneously/sequentially in sterile conditions.

Advantageously, the sealing machine may be performed under laminar flow and the working area is consequently advantageously in conformity with ISO 5.

Advantageously, the manufacturing process and system, from the production of the hematological tubes to their packaging into a double blister, allows manufacturing of tubes and kits for medical and surgical use in totally controlled aseptic conditions as well as bioburden controlled.

Current manual methods of preparing hematology tubes, PRP kits or A-PRP kits necessitate 4 machines, a number of technicians (approximately 10 technicians) and with an output of approximately 1500 PRP kits per week.

Advantageously, the methods and machines of the present invention only necessitate a small number of technicians (approximately 3 operators) and with a substantially higher output of approximately 2000 to 5000 PRP kits per day. The machines have been designed in order to be able to produce 5000 PRP kits per day.

In another aspect, the disclosure provides a method of manufacturing hematology tubes by means of a filling machine and/or a sealing machine according to any of the previous aspects.

In another aspect, the disclosure provides a method of automatically manufacturing hematology tubes by means of a filling machine comprising:

    a. filling the hematological tubes with either:

    i. an anticoagulant,
    ii. a polymer and an anticoagulant,
    iii. hyaluronic acid and/or chitosan and an anticoagulant, or
    iv. hyaluronic acid and/or chitosan, a polymer and an anticoagulant,

  b. optionally allowing controlled vacuum and clogging of the hematology tubes.

Other substances described herein may be combined during one or more of the steps of a manufacturing method of the disclosure.

In another aspect, the hematology tubes are not filled with any substances (tubes remain empty), but undergo controlled vacuum and clogging.

In another aspect, the disclosure provides a method of automatically manufacturing hematology tubes by means of a filling machine comprising controlled vacuum and clogging of the hematology tubes.

In another aspect, the disclosure provides a method of automatically manufacturing hematology tubes by means of a filling machine comprising:

  a. moving empty or prefilled hematology tubes one by one,
  b. filling the hematological tubes with either:

    i. an anticoagulant,
    ii. a polymer and an anticoagulant,
    iii. hyaluronic acid and/or chitosan and an anticoagulant, or
    iv. hyaluronic acid and/or chitosan, a polymer and an anticoagulant,

  c. optionally allowing controlled vacuum and clogging of the hematology tubes.

In another aspect, the disclosure provides a method of automatically manufacturing hematology tubes by means of a filling machine comprising:

  a. moving empty or prefilled hematology tubes one by one,
  b. filling the hematological tubes with a polymer,
  c. filling the hematological tubes with an anticoagulant,
  d. optionally allowing controlled vacuum and clogging of the hematology tubes.

In another aspect, the disclosure provides a method of manufacturing hematology tubes according to the previous claim comprising:

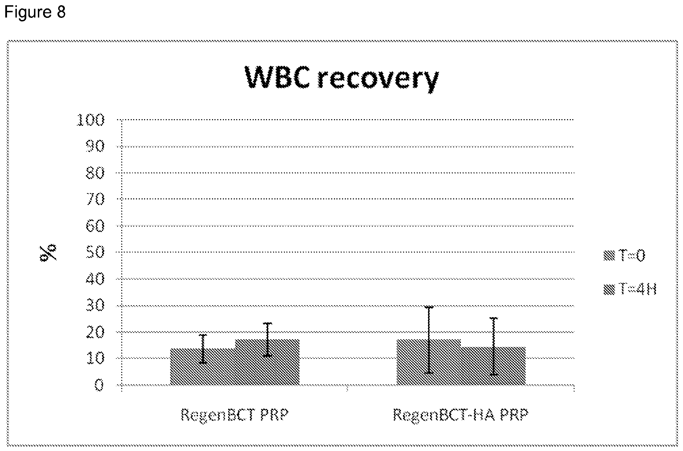

  a. loading empty or prefilled hematology tubes in empty pucks (Figure 2, 1) located at an counter clockwise (CCW) inlet rotary table (Figure 2, 2),
  b. inserting and entering of the pucks with the hematology tubes in a CCW rotating wheel (Figure 2, 3),
  c. moving the pucks to a first hot dose station (Figure 2, 4).
  d. injecting a polymer from a pressurized tank (Figure 2, 6) through hot hose (Figure 2, 5) inside the hematology tube,
  e. moving the hematology tubes to a second dosing station (Figure 2, 8),
  f. filling the hematology tubes with an anticoagulant through a nozzle and a peristaltic pump (Figure 2, 7),
  g. moving the filled hematology tubes to a Pick and Place (P&P) station (Figure 2, 9),
  h. inserting a stopper and a cap set coming from a vibrator (Figure 2, 10), through a rising rod in the pick arm of the P&P,
  i. moving back the hematology tubes to the rotary table, and
  j. collecting the filled and packed hematology tube by an operator.

In one aspect, the cap or plug is made of bromobutyl.

In another aspect, the disclosure provides a method of automatically manufacturing a PRP kit, A-PRP kit or medical device by means of a sealing machine for double blister packaging of hematological tubes with accessories comprising:

  a. molding, filling, sealing and cutting of a first blister comprising the hematological tubes with accessories,
  b. molding, sealing and cutting of a second blister on the first blister.

In another aspect, the disclosure provides a method of automatically manufacturing a PRP kit, A-PRP kit or medical device, comprising:

    a. moving empty or prefilled hematology tubes one by one,
    b. filling the hematological tubes with either:

        i. an anticoagulant,
        ii. a polymer and an anticoagulant,
        iii. hyaluronic acid and/or chitosan and an anticoagulant, or
        iv. hyaluronic acid and/or chitosan, a polymer and an anticoagulant,

    c. optionally allowing controlled vacuum and clogging of the hematology tubes,
    d. molding, filling, sealing and cutting of a first blister comprising the hematological tubes with accessories, and
    e. molding, sealing and cutting of a second blister on the first blister.

In another aspect, the disclosure provides a method of manufacturing a PRP kit, A-PRP kit or medical device comprising a method of manufacturing hematology tubes according to any of the previous aspects in combination with a method of manufacturing a PRP kit, A-PRP kit or medical device according to any of the previous aspects.

In another aspect, the disclosure provides a method of manufacturing a PRP kit, A-PRP kit or medical device by means of a filling machine according to any of the previous aspects and/or a sealing machine according to any of the previous aspects.

In one aspect, the hematology tubes are prefilled with a substance selected from agar, gelose, collagen, chitosan, growth factors, ascorbic acid, albumin, fibroin, silk-fibroin proteins or hyaluronic acid. Agar, gelose, collagen, ascorbic acid, albumin, silk-fibroin proteins may all display stabilizing and/or viscosity properties useful for the composition of the present invention. In one aspect, hyaluronic acid or chitosan may be substituted by or combined with agar, gelose, collagen, ascorbic acid, albumin, fibroin and/or silk-fibroin proteins. Preferably, hyaluronic acid or chitosan may be substituted by or combined with fibroin or silk-fibroin proteins. In one aspect, fibroin or silk-fibroin proteins may be combined with PRP. In another aspect, fibroin or silk-fibroin proteins may be combined with chitosan and/or HA in combination with PRP. In another aspect, albumin may be combined with PRP. In another aspect, albumin may be combined with chitosan and/or HA in combination with PRP. In another aspect, albumin may be combined with chitosan and/or HA, silk-fibroin proteins, and further combined with PRP.

In one aspect, a substance selected from agar, gelose, collagen, chitosan, growth factors, ascorbic acid, albumin, fibroin, silk-fibroin proteins or hyaluronic acid, and/or combinations thereof may be injected in the hematology tubes.

In one aspect, instead of or in combination with hyaluronic acid, a similar substance may be used or combined, for example gelose, agar, collagen chitosan, albumin and/or silk-fibroin proteins, and/or any combinations thereof.

Preferably, the anticoagulant is citrate or sodium citrate.

Preferably, the polymer is a thixotropic gel.

In one aspect, a syringe or a bowl may be used instead of a tube or hematology tube. In one aspect, any device that may be centrifuged may be used instead of a tube.

Preferably, the tube, syringe, kit or device is for human use or human treatment. In one aspect, the tube, syringe, kit or device may be used for animals, or adapted for veterinary use or animal treatment

Preferably, the method of manufacturing according to any of the previous aspects is performed under laminar flow and/or bioburden controlled.

Preferably, the machine according to any of the previous aspects is located under laminar flow and/or bioburden controlled conditions.

Preferably, the blister is made of APET or PETG film. Preferably sealing is made with a tyvek film.

The hematology tubes may be of different shapes nad made of crystal, plastic or metal. Preferably, the tubes are made of plastic, preferably COP or COC, preferably without phtalates.

The hematology tubes have preferably a capacity of about 9 to about 10 ml. Preferably, the tubes are about 11 to 13 cm in length and about 1.5 cm in width. For such a tube capacity, about 1 gr. to about 3 gr. of thixotropic gel may be added, preferably about 2 gr. of thixotropic gel. For such a tube capacity, about 1 gr. to about 3 gr. of sodium citrate may be added.

In another aspect, the disclosure provides an hematology tube, syringe, PRP kit, A-PRP kit or medical device obtained by a method according to any of the previous aspects or obtained by means of a machine according to any of the previous aspects.

The hematology tubes, syringe, kits and devices of the present disclosure may be used, but not limited to, thrombin, platelet concentrate, PRP composition, A-PRP composition, PRP kit, A-PRP kit and wound healant preparations.

In another aspect, the disclosure provides a use of a machine according to any of the previous aspects for the manufacture of hematology tubes, syringe, PRP kits, A-PRP kits or medical devices.

Advantageously, standardization is obtained by the manufacturing processes of the present disclosure. Advantageously, the hematology tubes obtained by the manufacturing processes of the present disclosure are more reliable in terms of design (design for human use), predictability of results, and replicability. The hematology tubes, syringe, PRP kits, A-PRP kits obtained by the manufacturing processes of the present invention have the following advantages in terms of:

i.) Efficacy as a high yield of PRP, A-PRP is obtained, i.e. cell counts per tube (high yield of cells, i.e. platelets and leukocytes),

ii.) Cell viability as the cells are less subject to traumatic stresses, the manufacturing processes of the present invention being designed as non-traumatic processes,

iii.) Cell functionality as high cell functionality is obtained,

iv.) Safety as the entire process is standardized with GMP compliancy, performed under laminar flow, and compliant with various ISO norms such as ISO14644-1, ISO14644-3, ISO12469, ISO11607 (1 and 2). High quality standards for the machines and the manufacturing processes are implemented.

Advantageously, the automated double blistering is in conformity with ISO norms ISO11607 (1 and 2).

Combined with the high quality standards of the PRP or autologous PRP preparations processes, the compositions of the present disclosure are particularly adapted and useful in therapy, cosmetics, surgery and other applications herein described.

Furthermore, the current manual manufacturing processes of PRP kits are fastidious, time-consuming and necessitate the intervention of various human resources.

The automated manufacturing processes for hematological tubes, A-PRP kits and PRP kits of the present disclosure are able to provide very good output, to increase the quality of the A-PRP or PRP preparations in terms of efficacy, cell viability and cell functionality, and at the same time to provide hematological tubes, A-PRP kits and PRP kits of high quality standards with constant predictable and reliable results and meeting high safety standards.

The present disclosure also relates to hyaluronic acid and/or chitosan formulations alone or combined with a platelet concentrate, preferably platelet rich plasma (PRP) suitable for human use. These compositions may also be suitable for veterinary use.

In another aspect, the disclosure provides a composition comprising hyaluronic acid (HA) of about 1000 KDa to about 2000 KDa at about 1.5% to about 2.5% concentration. Preferably, the disclosure provides a composition comprising hyaluronic acid of about 1400 KDa to about 1600 KDa at about 1.8% to about 2.2% concentration. More preferably, the disclosure provides a composition comprising hyaluronic acid of about 1550 KDa at about 1.8% to about 2.2% concentration, more preferably from about 1.7% to about 2% concentration. Such compositions are particularly adapted for injections or infiltrations, intra dermal injections, subcutaneous applications, intra-articular infiltrations, fistulas and/or as a biological glue.

Such compositions of hyaluronic acid of are also particularly adapted for a combination with a platelet concentrate, preferably a platelet rich plasma (PRP), see Examples. For proper manipulation of PRP combined with hyaluronic acid, specific formulations of HA are required as hyaluronic acid outside a specific molecular weight and concentration will be too difficult to manipulate and not adapted for industrial application. It has been found that a molecular range of about 1400 KDa to about 1600 KDa at about 1.8% to about 2.2% concentration is adapted for use with PRP. These specific properties of hyaluronic acid are important in order to maintain suitable viscosity. Appropriate viscosity of hyaluronic acid is required for its manipulation, for example in a syringe. Advantageously, the HA solution is a homogenous viscous solution adapted for combination or mixing with a platelet concentrate, preferably PRP. The present hyaluronic acid formulations are therefore particularly suitable for industrial application as a specific range of HA molecular weight and concentration permit combination with a platelet concentrate, for example PRP.

In another aspect, the inventions provides a composition comprising hyaluronic acid of about 1400 KDa to about 1600 KDa at about 1.8% to about 2.2% concentration and a platelet concentrate, preferably platelet rich plasma.

In another aspect the invention provides a composition comprising hyaluronic acid of about at least 4000 KDa (about 4000 Kda or above 4000 KDa) at about 1.5% to about 2.5% concentration. Preferably, the invention provides a composition comprising hyaluronic acid of about at least 4000 KDa (about 4000 KDa or above 4000 KDa) at about 1.8% to about 2.2% concentration, more preferably of about 4000 KDa at about 2% concentration. In one embodiment, the invention provides a composition comprising hyaluronic acid of about 4000 KDa to about 6000 KDa at about 1.8% to about 2.2% concentration. Such compositions are particularly adapted for mechanical support, for intra dermal injections, subcutaneous applications, volumetric corrections and or visco-supplementation.

In one embodiment, the present invention encompasses a combination of at least two hyaluronic acids differing in molecular weight and in concentration. Having two HAs only differing in molecular weight is not sufficient, an appropriate concentration for each HA is crucial in order to be able to manipulate the combination of hyaluronic acid, for example with a syringue (see Examples 1 to 3).

In another aspect, the invention provides a hyaluronic acid composition comprising at least one low molecular weight hyaluronic acid and at least one high molecular weight hyaluronic acid characterized in that:

- the low molecular weight hyaluronic acid is less than 600 KDa or about 600 KDa, and
- the high molecular weight hyaluronic acid is about 4000 Kda or above 4000 KDa (about at least 4000 KDa).

In another aspect, the invention provides a hyaluronic acid composition comprising one low molecular weight hyaluronic acid and one high molecular weight hyaluronic acid characterized in that:

- the low molecular weight hyaluronic acid is less than 600 KDa or about 600 KDa, and
- the high molecular weight hyaluronic acid is about 4000 Kda or above 4000 KDa (about at least 4000 KDa).

At least one hyaluronic acid has a molecular weight of less than 600 KDa or a molecular molecular weight of about 600 KDa, and at least one hyaluronic acid has a molecular weight of more than 4000 KDa or a molecular molecular weight of about 4000 KDa. In one embodiment, at least one hyaluronic acid has a molecular weight of about 400KDa to about 600 KDa, and at least one hyaluronic acid has a molecular weight of about 4000 KDa to about 6000 KDa.

HA may be reticulated or non reticulated.

Preferably, the respective ratio is about 2:3 (low molecular weight hyaluronic acid to high molecular weight hyaluronic acid; i.e. hyaluronic acid of less than 600 KDa or about 600 KDa to hyaluronic acid of more than 4000 KDa or about 4000 KDa) with a total concentration of about 2.2% to about 2.8%. Alternatively, the respective ratio is about 8:5 with a total concentration of about 3% to about 3.5%. Such formulations are particularly adapted for mechanical support, for intra dermal injections, subcutaneous applications, volumetric corrections and or visco-supplementation. Advantageously, such hyaluronic acid combinations procure suitable viscosity for manipulation (see Examples 1 to 3). Advantageously, in the combination of HA differing in molecular weight, a high molecular weight HA may increase viscosity suitable for mechanical support and a low molecular weight HA may contribute to cell proliferation/regeneration by protecting cells and their activity.

In another aspect, the disclosure provides a composition or a device, preferably a tube or syringe, comprising hyaluronic acid, preferably a HA composition of the present invention and optionally chitosan.

In another aspect, the disclosure provides a composition or a device, preferably a tube or syringe, comprising hyaluronic acid, preferably a HA composition of the present invention and a platelet concentrate, preferably PRP, more preferably a PRP of the present disclosure.

In one aspect, the disclosure provides a composition or a device, preferably a tube or syringe, comprising hyaluronic acid, preferably a HA composition of the present invention, chitosan and a platelet concentrate, preferably PRP, more preferably a PRP of the present disclosure

In one aspect, the disclosure provides a composition or a device, preferably a tube or syringe, comprising chitosan and a platelet concentrate, preferably PRP, more preferably a PRP of the present disclosure. Chitosan may be particularly suitable in a combination with HA and/or a platelet concentrate for its stabilizing properties and viscosity maintenance. Chitosan may further enhance the stability and/or efficiency of a PRP-HA composition. Moreover, chitosan has coagulation properties at physiological temperature, i.e. around 37°C. The coagulation properties are absent at room temperature making chitosan particularly useful for human use. Advantageously, no other coagulation activator than chitosan may be required in a formulation of the present invention. Chitosan may therefore not only be useful for its stabilization and viscosity maintenance properties, but also as coagulation activator. Chitosan may therefore be particularly useful for all formulations requiring a coagulation activator as for the preparation of platelet rich plasma. The presence of chitosan makes such formulation particularly suitable for all indications or treatments involving the cartilage. In one embodiment of the present disclosure, a coagulation activator of the present invention may be substituted or combined with chitosan.

In another aspect, the disclosure provides a composition or a device, preferably a tube or syringe, comprising hyaluronic acid, preferably a HA composition of the present invention and an anticoagulant.

In one aspect, the disclosure provides a composition or a device, preferably a tube or syringe, comprising hyaluronic acid, preferably a HA composition of the present invention, chitosan and an anticoagulant.

In one aspect, the disclosure provides a composition or a device, preferably a tube or syringe, comprising chitosan and an anticoagulant.

In another aspect, the disclosure provides a composition or a device, preferably a tube or syringe, comprising hyaluronic acid, preferably a HA composition of the present invention and a cell selector gel.

In one aspect, the disclosure provides a composition or a device, preferably a tube or syringe, comprising hyaluronic acid, preferably a HA composition of the present invention, chitosan and a cell selector gel.

In one aspect, the disclosure provides a composition or a device, preferably a tube or syringe, comprising chitosan and a cell selector gel.

In another aspect, the disclosure provides a composition or a device, preferably a tube or syringe, comprising hyaluronic acid, preferably a HA composition of the present invention and an anticoagulant, and a cell selector gel.

In one aspect, the disclosure provides a composition or a device, preferably a tube or syringe, comprising hyaluronic acid, preferably a HA composition of the present invention, chitosan and an anticoagulant, and a cell selector gel.

In one aspect, the disclosure provides a composition or a device, preferably a tube or syringe, comprising chitosan and an anticoagulant, and a cell selector gel.

In another aspect, the disclosure provides a device, preferably a tube or syringe, comprising hyaluronic acid and/or chitosan and whole blood or a platelet concentrate, preferably PRP.

In another aspect, the disclosure provides a device, preferably a tube or syringe, comprising hyaluronic acid and/or chitosan, an anticoagulant and whole blood or a platelet concentrate, preferably PRP.

In another aspect, the disclosure provides a device, preferably a tube or syringe, comprising hyaluronic acid and/or chitosan, a thixotropic gel and whole blood or a platelet concentrate, preferably PRP.

In another aspect, the disclosure provides a device, preferably a tube or syringe, comprising hyaluronic acid and/or chitosan, an anticoagulant, a cell selector gel and whole blood or platelet concentrate, preferably PRP.

In another aspect, the disclosure provides a composition according to the invention further comprising whole blood, a platelet concentrate, platelet rich plasma and/or chitosan.

In another aspect, the disclosure provides a device, preferably a tube or syringe, comprising a composition according to the invention and/or chitosan, and further optionally comprising an anticoagulant and/or a cell selector gel. HA or chitosan may herein be substituted or combined with fibroin and/or silk-fibroin proteins.

In another aspect, the disclosure provides a medical device or kit comprising a device, tube or syringe according to the invention.

The device may be a syringe, bowl or tube, including any device which may be centrifuged.

Preferably, the hyaluronic acid is a HA composition of the present invention. Preferably, the platelet concentrate or PRP is a platelet concentrate or PRP of the present disclosure.

Preferably, the anticoagulant is sodium citrate. Preferably, the cell selector gel is a thixotropic gel. Preferably, hyaluronic acid is located at the bottom of the tube or syringe, followed by a thixotropic gel and above an anticoagulant, preferably sodium citrate.

The anticoagulant of the present invention may be citrate, for example a buffered sodium citrate solution at 0.10 M or an anhydrous sodium citrate at 3.5 mg/mL. Preferably, sodium citrate is at about 0.109M.

In a preferred aspect, the disclosure provides a device, preferably a tube, comprising about 2.0 g of hyaluronic acid 1550KDa, about 1.9 g of polymer gel (thixotropic gel), and about 0.7ml sodium citrate solution at about 0.109M.

Preferably, the device, preferably a tube, contains about 1 ml to about 2 ml of hyaluronic acid, about 2g of cell selector or thixotropic gel and about 1ml of sodium citrate at 0.109M.

Advantageously, such formulations are particularly adapted for industrial application and human use having (see Examples 1 to 3):

- good stability at room temperature,
- adequate viscosity with good manipulability being suitable for use with a syringe and needle (flow tests),
- no adjuvant like mannitol required as for example with osteonil,
- presence of a high molecular weight HA procures stability and has a long-lasting effect,
- presence of a low molecular weight HA may favor cellular growth and will be metabolized more rapidly, with a faster action on the visco supplementation (high penetration).

HA formulations and/or chitosan may be further combined with a cell extract. Enriched media may be used.

Such formulations prove useful when new cells are required, for example when the muscle necessitates fibroblasts.

In one aspect, an HA formulation of the present invention and/or chitosan may be combined with a platelet concentrate and T.C.P. (TriCalcium-Phosphate).

In further aspects, the disclosure provides a device, preferably a tube, which may be used for the preparation of a wound healant composition or tissue healant composition, selected from:

i) a glass separator tube comprising hyaluronic acid and/or chitosan, a polyester-based thixotropic gel and a buffered sodium citrate solution at 0.10 M,
ii) a polyethylene terephthalate separator tube comprising hyaluronic acid and/or chitosan, a highly thixotropic gel formed by a polymer mixture and an anhydrous sodium citrate at 3.5 mg/m,
iii) a Cyclic Olefin Copolymer (COC) or Cyclic Olefin Polymer (COP) separator tube comprising hyaluronic acid and/or chitosan, a polyester-based thixotropic gel and a buffered sodium citrate solution at 0.10 M, or
iv) a Cyclic Olefin Copolymer (COC) or Cyclic Olefin Polymer (COP) filter separator tube containing hyaluronic acid and/or chitosan, and a buffered sodium citrate solution at 0.10 M or an anhydrous sodium citrate at 3.5 mg/mL.

Preferably, the HA formulation is mixed or concentrated, preferably at about 2%, in phosphate buffer (PBS).

For industrial application, hyaluronic acid must undergo sterilization. It has surprisingly been found that gamma irradiation is unsuitable for hyaluronic acid (see Examples). Gamma irradiation renders hyaluronic acid inactive by denaturing hyaluronic acid. The hyaluronic acid formulations of the present invention have to undergo a specific procedure in order to maintain their properties. Only steam sterilization has been determined to be suitable for HA sterilization maintaining the properties and activity of hyaluronic acid (see Examples). Steam sterilization is required in order to maintain HA viscosity and functionality as shown in the Examples.

In another aspect, the HA formulations of the present invention are steam sterilized. In one embodiment, the HA formulations are steam sterilized from about 105°C for about 8 minutes, to about 121°C for about 20 minutes. Preferably, HA is sterilized by steam at about 105°C for about 8 minutes. In a preferred embodiment, the device or tube comprising the HA formulation is steam sterilized.

In another aspect, the present disclosure provides a wound healant or tissue healant comprising a composition according to the invention.

In another aspect, the present disclosure provides a composition according to the invention for use in therapy.

In another aspect, the present disclosure provides a method or process for the preparation of a wound healant or tissue healant comprising a platelet concentrate composition or platelet-rich plasma composition, comprising the steps of:

> a) Centrifuging whole blood in a device, preferably a tube or syringe, comprising a composition according to the invention, preferably a HA composition, chitosan composition or a HA and chitosan composition, and
> b) Collecting the wound healant or tissue healant comprising a platelet concentrate composition or platelet-rich plasma composition.

Preferably, the centrifugation step is performed at a force of or about 1500g up to about 2000g (this speed is with a radius of about 20 cm at about 2500 to about 3000 rpm). Preferably, the centrifugation step is performed in a sufficient length of time to form a barrier between the plasma containing the platelets, the lymphocytes and the monocytes and the gel containing the erythrocytes. Preferably, centrifugation time is about 3 minutes. In one preferred embodiment, centrifugation speed is about 1500 g with centrifugation time of about 5 minutes. Centrifugation time and speed depends on the formulation present in the device. The skilled artisan can determine the appropriate centrifugation time and speed according to the composition used.

In one aspect, the wound healant or tissue healant is separated from the full plasma by removing first about half of the supernatant containing the platelet poor plasma.

Optionally, after the centrifugation step, the platelet concentrate or platelet rich plasma is mixed with the hyaluronic acid and/or chitosan.

Optionally, after the centriguation step, the device or tube is homogenized by gently inverting the device or tube several times, resulting in re-suspending the cellular deposit in the supernatant and/or homogenizing the hyaluronic acid and/or chitosan with PRP. From about 5 ml starting material (whole blood) in a tube, about 4 ml to about 5 ml of the mix PRP - Hyaluronic acid/chitosan will be obtained for each tube, of which about 2 to about 3 ml of anticoagulated plasma combined with about 2 ml of hyaluronic acid/chitosan solution contained in the device. The final volume of the PRP-HA mix is around 4 to around 5 ml, with an approximate ratio of PRP to hyaluronic acid/chitosan solution of 50%.

Optionally, a cannula can be used in order to initiate the homogeneization. In order to obtain a higher cellular concentration, or a higher viscosity of the mix, before proceeding to the platelet resuspension, about 1 ml of the upper layer of the platelet poor plasma (PPP) supernatant may be delicately removed with a long cannula. Resuspending the cellular deposit in the remaining PRP by gentle inversions of the tube may then be performed (homogenization).

In another aspect, the invention provides a process or method for the preparation of a wound healant composition or tissue healant composition comprising a platelet concentrate composition or platelet-rich plasma composition, comprising the steps of:

> a) Centrifuging whole blood in a device, preferably a tube or syringe comprising a composition according to the invention, preferably a HA composition, a chitosan composition or a HA and chitosan composition,
> b) Optionally removing platelet poor plasma, preferably about 1 ml of the upper layer of the platelet poor plasma,
> c) Optionally homogenizing the resulting composition and/or re-suspending the cellular deposit in the supernatant, preferably by inverting the device, tube or syringe,
> d) Collecting the wound healant or tissue healant comprising a platelet concentrate composition or platelet-rich plasma composition,

e) Optionally further mixing said wound healant or tissue healant,

f) Optionally further combining said wound healant or tissue healant with at least one additional substance and/or one or more cell extracts.

In another aspect, the present disclosure provides a wound healant or tissue healant composition prepared according to a method of the present disclosure.

Preferably, a device, preferably a tube or syringe, according to the disclosure is used in a method or process according to the disclosure. Preferably, the device, tube or syringe does not contain phthalate.

In one embodiment, the wound healant or tissue healant may be combined with at least one additional substance such as a coagulation activator, thrombin serum, tricalcium phosphate (TCP), a bone substitute, calcium gluconate, calcium saccharate, chitosan, fibroin, silk-fibroin proteins, growth factors, mannitol, collagen, albumin, ascorbic acid, cream, fat cells, fat tissue, bone marrow concentrate, lubricin, cd-gelatin, botulinum toxin and/or one or more cell extracts, which are not embryonic stem cell extracts preferably an autologous cell extract, preferably a cell extract selected from an extract of keratinocytes, bone marrow, fibroblasts, periosteum or corneal cells, melanocytes and Langheran's cell; fat cells, muscle cells such as myoblasts and satellite cells; osteoblasts; chondrocytes; umbilical cord cells; non embryonic stem cells, mesenchymal stem cells (MSCs), preadipocytes, pre-endhotelial cells, Schwann cells, glial cells, neurones or Achilles tendon cells.

In one disclosure, calcium gluconate at about 1% to about 10% may be added for appropriate coagulation of the infected site. In one disclosure, calcium chlorure may be used. More preferably, calcium gluconate at about 10% may be used. Alternatively, calcium saccharate may be used. In one aspect, a combination of calcium gluconate and calcium saccharate may be used. For example, for a 100 ml of solution, about 9.5g of Calcium gluconate and about 360mg of Calcium saccharate may be used. For example, for a 2 ml single dose ampoule about 0.19 g of calcium gluconate and about 7.2 mg calcium saccharate may be used for a calcium content of about 0.463 mmol per 2 ml ampoule. For example, for a 5 ml single dose ampoule, about 0.47 g of calcium gluconate and about 18 mg calcium saccharate may be used for a calcium content of about 1.148 mmol per 5 ml ampoule. The skilled artisan will easily determine the appropriate calcium content according to the specific use.

The HA formulations of the present invention provide a volume effect, with desirable long term outcome on skin. Cells are being smoothed or flattened. Fibroblasts are stimulated, the activity of fibroblasts are being protected. The HA formulations of the present invention allows maintenance of fibroblast's structure.

Formulations may be adapted to a specific use. For injection and/or infiltration, the formulations may be used without additional substance as a viscous formulation.

As other useful applications to the skilled artisan/surgeon, a biological glue may be obtained with PRP, A-PRP or PRP in combination with HA without additional substances. The dispensation requires the use of a dual dispenser that allow the formation of the glue when the PRP plus HA substances are injected simultaneously into the surgical site. In one embodiment, a dual dispenser may be used for the injection of a formulation of the present invention.

In order to obtain a stronger biological glue, the HA-PRP formulations of the present disclosure may be combined with a coagulation activator like a calcium salt, preferably CaCl2, or thrombin, preferably an autologous thrombin. Alternatively or in addition to CaCl2 or thrombin, the HA-PRP formulations of the present disclosure may be combined with calcium gluconate and/or calcium saccharate.

For other applications evident to the skilled artisan, a suturable membrane may be more suitable. In order to obtain a suturable membrane, the HA-PRP formulations of the present disclosure may be combined with a higher concentration of coagulation activator like an autologous or homologous thrombin, a calcium salt like CaCl2, but preferably Calcium Gluconate. Alternatively or in addition to CaCl2 or thrombin, the HA-PRP formulations of the present disclosure may be combined with TCP (TriCalcium Phosphate). The HA-PRP formulations of the present disclosure may be combined with TCP for deep injection and/or volume enhancement.

For application of the HA-PRP formulations of the present disclosure, several means may be used by the skilled artisan. For example, a dual applicator may be used. Hyaluronic acid may be present in one device, for example a tube or syringe, and PRP may be present in another device, for example a tube or syringe. Alternatively, the HA-PRP formulations may be prepared in one single device, for example a tube or syringe.

HA alone or a combination of HAs may be further combined with at least one cell extract, provided the cell is not an embryonic stem cell. HA alone or a combination of HAs may be further combined with chitosan, collagen or albumin. Chitosan may be form animal origin, for example crustacean, or vegetal origin, for example Paris mushroom.

Compositions of the present disclosure may be used as ophthalmic collyre (eyewash), in articulations (for example knee), sports medicine, muscular lesions or rotator cuff.

Compositions of the present disclosure may be used in esthetics, mesotherapy, wrinkle filling (superficial and profound), as a mask post laser, post peeling or monotherapy (for example glitter, gloss, brilliance or brightness) or erectile dysfunction.

Compositions of the present disclosure may be used in wound care, diabetic wounds or in large vascular wounds.

Compositions of the present disclosure may be used in ophthalmology.

The methods of the present disclosure permit the preparation of a composition, wound or tissue healant ready to be used in approximately 5 minutes.

The compositions of the present disclosure have been tested for biocompatibility, toxicity and for implantations tolerance (data not shown). Advantageously, the compositions of the present invention are suitable for industrial application as all the biocompatibility studies and toxicity tests have a favorable outcome. The compositions have for example no adverse effects on rabbits and implants are resorbed in 30 days in the majority of sites. The HA compositions have also been tested for molecular weight, viscosity and concentration after accelerated aging at 50°C (data not shown). No notable degradation compound has been identified. Further tests made include cytotoxicity, irritation, sensitivity, reverse mutations, hemolysis and thromboplastin tests.

In another aspect, the present disclosure encompasses tubes and/or syringes comprising the formulations of the present invention.

The formulations of the present disclosure may be combined, stored or delivered in tubes and/or syringes.

In another aspect, the present disclosure encompasses a kit or medical device for the preparation of a formulation according to the present invention.

The HA and/or chitosan compositions may be combined and/or administered in several ways. In one aspect of the disclosure, an composition according to the invention may be combined or mixed with PRP in a procedure as described in WO2011/10948. In one aspect, the HA and/or chitosan formulation is present in a tube and the platelet concentrate or PRP is prepared using the same tube. The PRP and HA/chitosan may then be mixed together following centrifugation. Alternatively, the PRP and HA/chitosan formulation is combined or mixed together in another device, for example a syringe. The HA/chitosan, instead of being present in a tube, is inserted in a syringe which will thereafter also be used for the preparation of PRP. The disclosure encompasses therefore all devices wherein an HA/chitosan may be inserted and wherein the same device is used for the preparation of a platelet concentrate. Preferably, the HA/chitosan and PRP are being prepared and mixed together using only one device. In one disclosure, the HA/chitosan and PRP are being prepared, mixed together and applied on the patient (for example by injection) using only one device. HA/chitosan means either a HA composition alone, a chitosan composition alone or a combination of HA and chitosan. For example, in another disclosure, chitosan is added or mixed to the HA-PRP composition.

Alternatively, the HA/chitosan formulation and the platelet concentrate (for example PRP) are being prepared in two separate devices or more, whether tube, syringe or else. Once prepared, the HA/chitosan and PRP may be mixed together with the use of a connector or other device, or being transferred to another device.

Formulations comprising HA and PRP or PRP alone were tested for their properties and performance using two devices in parallel called RegenBCT tube for PRP alone preparation and RegenBCT-HA tube for a preparation combining PRP and hyaluronic acid (Example 3). Alternatively to RegenBCT tubes, A-CP tubes may be used in the present invention, for example to be combined with HA in order to get A-CP-HA tubes. A-CP tubes, like RegenBCT tubes. may therefore be prefilled with HA, chitosan and/or fibroin or fibroin-silk proteins. Advantageously, the formulations comprising HA and PRP in comparison to PRP alone showed:

- identical pH,
- equivalent results in removing red blood cells with a recovery inferior to 0.5%,
- equivalent reduction of the white blood cells content in PRP, compared to whole blood with around 85% of the leukocytes removed and with same repartition in white blood cells subsets with a low contamination of granulocytes, known to be proinflammatory,

- a platelet concentration higher than the blood platelet concentration,
- a similar pattern of answer in the two kind of platelet preparations for selectin expression, with an increase of P selectin expression in response to specific activation with ADP,
- a response to hypotonic stress that was of the same amplitude in both types of platelet preparations and remained unchanged during the period of 4 hours, and
- ability to aggregate in response to collagen activation and this ability was maintained at the same level at time T=4 hours.

More specifically, the PRP obtained with the RegenBCT-HA tube has a high platelet recovery (60-70%), a partial recovery of white blood cells (around 15%), with a selective depletion (95%) of granulocytes and almost no red blood cell contamination (<0.5%).

Surprisingly, an increased number of platelets was found in the RegenBCT-HA PRP after four hours which may be due to the release in the plasma of some platelets from the acid hyaluronic fraction during the length of the experiment. The HA-PRP formulations of the present disclosure may therefore have a long-lasting effect as platelets are being released with time. Of importance, a too high concentration of platelets may have an inhibitory effect. Progressive release of platelets using the compositions, devices and methods of the present disclosure represents an important advantage.

Advantageously, the resulting HA-PRP formulation is a platelet suspension in a three dimensional scaffold (HA) with a concentration near the physiological blood value, in which the platelets have maintained their integrity and functionality.

In another aspect, the disclosure provides HA-PRP formulations characterized in that:

- red blood cells concentration of less than $0.5X\ 10^6/mm^3$, preferably less than $0.3X\ 10^6/mm^3$, more preferably less than $0.1X\ 10^6/mm^3$
- almost no red blood cell contamination with a red blood cell recovery of about $0.49 \pm 0.25$ %
- white blood cell recovery of about $14.56 \pm 10.66$ %
- repartition of granulocytes at time T=0 is about 18%, at time 4 hours is about 23%, repartition of mononuclear cells at time T=0 is about 82% and at time 4 hours is about 77%
- selective depletion of about 95% granulocytes
- recovery of mononuclear cells is about $37.87 \pm 25.17$ %
- recovery of granulocytes is about $5.48 \pm 4.92$ %
- final platelet concentration factors in the PRP-HA mixes were about $0.71 \pm 0.14$ and about $0.85 \pm 0.17$ respectively at time T=0 and four hours later
- final platelet concentration factors with RegenBCT-HA PRP were about $1.53 \pm 0.36$ and about $1.86 \pm 0.45$ respectively at time T=0 and four hours later
- recovery of platelet is about $59.43 \pm 14.48$ % at time T=0 and about $71.70 \pm 15.14$ % four hours later
- starting volume of about 5ml to about 5.5ml whole blood, about 2.4 PRP volume, about 2.7 removed volume and about 5.2 ml of PRP-HA
- pH value of about 7.7

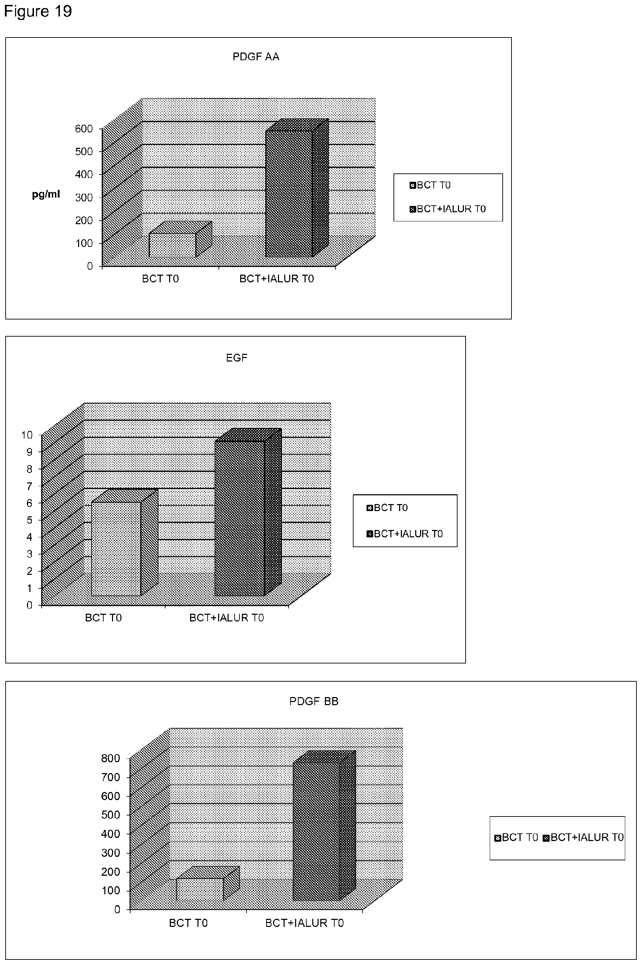

Efficacy of the HA-PRP formulations has been assessed in patients comparing PRP formulations using the REGENBCT tube and HA-PRP formulations using the REGENBCT-HA tubes (see Example 4). As shown in Figure 19, evidence of HA and growth factors (GF) affinity is demonstrated for PDGF and EGF. A much stronger GF release is observed in a combination of HA with PRP than PRP alone.

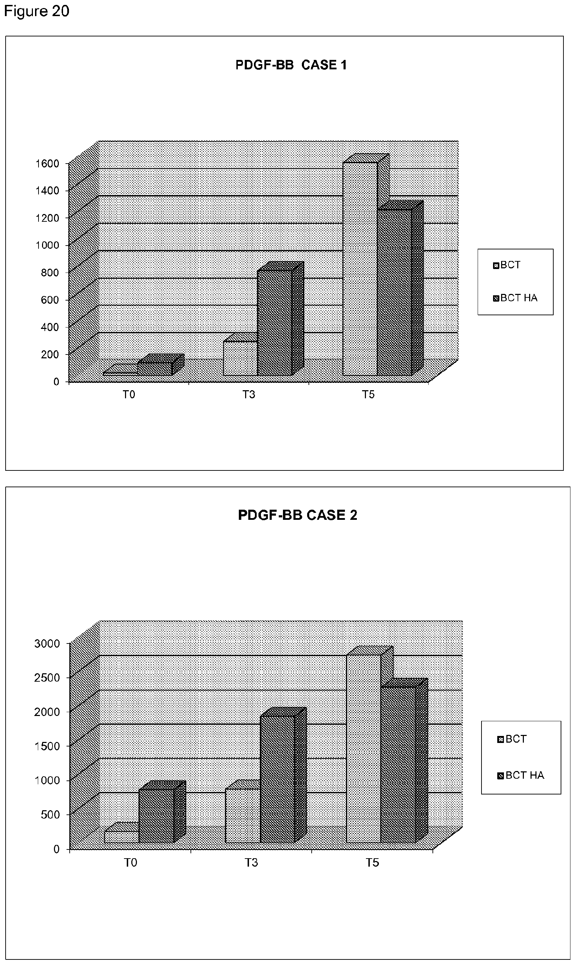

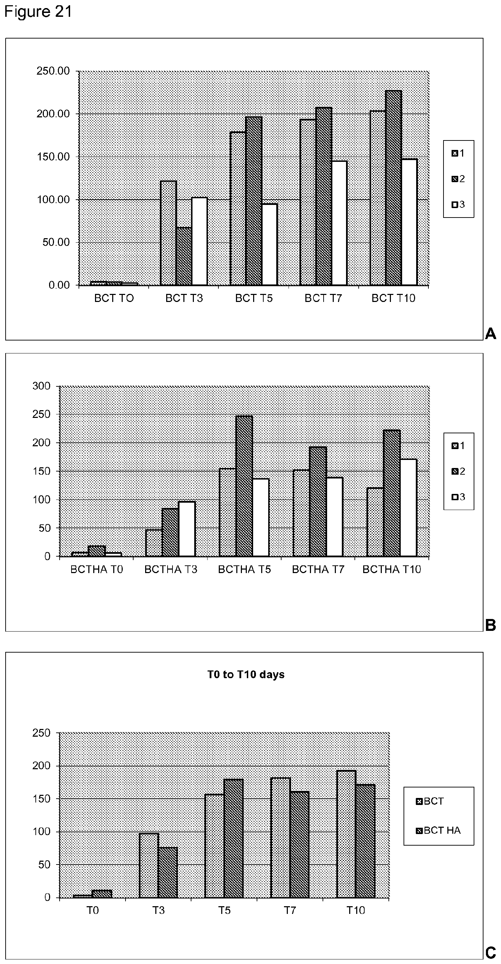

Surprisingly, the early phase GF delivery (PDGF-AA, PDGF-BB and EGF) is distinct between a PRP preparation alone (BCT) versus a combination of PRP and HA (BCT HA), see Figures 20 and 21.

A combination of PRP and HA is very effective in the early phase with much higher GF release, particularly for PDGF-BB until about day 3 or day 4 and for EGF until about day 1 or day 2. This is particularly important in all instances where a rapid and strong release of GF is required, for example in wound care. Advantageously, such early phase GF release enhances wound healing time and rapidly decreases pain. For example, a HA-PRP formulation according to the invention is useful in acute pain. The present HA-formulations may therefore also have an early-phase effect.

In another aspect, the disclosure provides a wound healant composition, tissue healant composition, cell composition, A-PRP composition, PRP composition, thrombin serum, or haemostatic agent obtained by using a hematology tube, syringe, A-PRP kit, PRP kit or medical device according to any of the previous aspects, obtained by using a machine according to any of the previous aspects or obtained by a method according to any of the previous aspects, wherein the wound healant composition, tissue healant composition, cell composition, platelet rich plasma composition, A-PRP composition or haemostatic agent is combined with a coagulation activator, thrombin serum, tricalcium phosphate (TCP), a bone substitute, calcium gluconate, calcium saccharate, chitosan, fibroin, silk-fibroin proteins, growth factors, mannitol, collagen, albumin, ascorbic acid, cream, fat cells, fat tissue, bone marrow concentrate, lubricin, cd-gelatin, botulinum toxin and/or one or more cell extracts, which are not embryonic stem cell extracts, preferably an autologous cell extract, preferably a cell extract selected from an extract of keratinocytes, bone marrow, fibroblasts, periosteum or corneal cells, melanocytes and Langheran's cell; fat cells, muscle cells such as myoblasts and satellite cells; osteoblasts; chondrocytes; umbilical cord cells; non-embryonic stem cells, mesenchymal stem cells (MSCs), preadipocytes, pre-endhotelial cells, Schwann cells, glial cells, neurones or Achilles tendon cells.

In one aspect, a further apparatus enables the automatic combination of a PRP composition or A-PRP composition with a coagulation activator, thrombin serum, tricalcium phosphate (TCP), a bone substitute, hyaluronic acid composition, calcium gluconate, calcium saccharate, chitosan, fibroin, silk-fibroin proteins, growth factors, mannitol, collagen, albumin, ascorbic acid, cream, fat cells, fat tissue, bone marrow concentrate, lubricin, cd-gelatin, botulinum toxin and/or one or more cell extracts, which are not embryonic stem cell extracts.

In one aspect, a coagulation activator, thrombin serum, cell selector gel, tricalcium phosphate (TCP), a bone substitute, hyaluronic acid composition, calcium gluconate, calcium saccharate, chitosan, fibroin, silk-fibroin proteins, growth factors, mannitol, collagen, albumin, ascorbic acid, cream, fat cells, fat tissue, bone marrow concentrate, lubricin, cd-gelatin, botulinum toxin and/or one or more cell extracts, which are not embryonic stem cell extracts, are prefilled, injected or inserted in the hematology tubes.

In another aspect, the disclosure provides a wound healant composition, tissue healant composition, cell composition, composition, platelet concentrate composition, HA composition, chitosan composition, PRP composition, A-PRP composition, thrombin serum, or haemostatic agent obtained by using a hematology tube, syringe, A-PRP kit, PRP kit or medical device according to any of the previous aspects, obtained by using a machine according to any of the previous aspects or obtained by a method according to any of the previous aspects for use in dentistry, orthopedics, sports medicine, cosmetics, esthetics, surgery, ophthalmology and/or mesotherapy.

In another aspect, the disclosure provides the use of a wound healant composition, tissue healant composition, cell composition, platelet concentrate composition, HA composition, chitosan composition, PRP composition, A-PRP composition, thrombin serum, or haemostatic agent obtained by using a hematology tube, syringe, A-PRP kit, PRP kit or medical device according to any of the previous aspects, obtained by using a machine according to any of the previous aspects or obtained by a method according to any of the previous aspects for cellular regeneration, for tissue adhesion, for promoting wound healing or tissue healing and/or sealing and/or regeneration of a tissue and/or a cartilage and/or a bone and/or a nerve in a wound or tissue of a human or animal, or for inducing periodontal regeneration in a wound or a periodontal defect of a mammal with periodontal disease or other condition requiring periodontal regeneration, or for ligament and/or cartilage reconstitution, or for promoting skin regeneration in a scar or a wrinkle, or for increasing adipose tissue volume in a mammal with a dermal fat graft or other condition requiring adipose tissue regeneration, or for inducing myocardial regeneration in a mammal with myocardial deficiency or other condition requiring myocardial regeneration tissue regeneration, or for inducing corneal regeneration in a mammal with corneal deficiency or other condition requiring corneal regeneration, or for inducing articular or cartilage regeneration in a mammal with articular or cartilage deficiency or other condition requiring articular or cartilage tissue regeneration, or for promoting skin regeneration in a scar, a wrinkle or a fat deficiency from human or lower animal, or for inducing peripheral nerve regeneration in a mammal with peripheral nerve damage, nerve suture or spinal cord injury or other condition requiring peripheral nerve regeneration, or for inducing bone regeneration in a mammal with bone damage, bone deficiency or other condition requiring bone regeneration, or for injections for orthopedic and injections for esthetic, or for regeneration and/or rejuvenation of skin tissues, particularly in promoting and/or initiating skin regeneration such as reducing skin wrinkles, deep wrinkles, acne, burns, rubella or small pox scars, vitiligo and lipoatrophy, amelioration of nasolabial lines and treatment of skin damages or disorders such as skin burns, Kaposi's sarcoma, skin skeloids or Dupuytren's palmar fibromatosis and in the reduction of pain associated with skin and tissue regeneration, or for wound or tissue healing or regeneration treatments, especially the treatment of traumatic or surgical wounds such in the fitting and/or holding and/or sealing of native or prosthetic grafts; treatment of vasculitis; ulcers such as diabetic neuropathic ulcers or decubitus sores, diabetic ulcer, perforating ulcer or diabetic perforating ulcer, arthritis, osteoarthritis, pseudo-arthritis, radiodermatitis and closing fistulas, or for cardiac disorders, cardiac regeneration such as in the treatment of heart failure, chronic cardiac failure, ischemic and non-ischemic cardiac failure and cardiomyopathy, or for bone, cartilage and articular disorders such as chondral damage, cartilage and/or bone injury such as deep cartilage damage and/or erosion and/or arthroscopy, tendon torn and rotator cuff in shoulder, or for corneal disorders such as dry eye syndrome; corneal opacity such as those caused by chemical burns, affliction by Steven's Johnson syndrome; scarring of the cornea and corneal ulcers, or for peripheral nerve damage, nerve suture and spinal cord injury.

In another aspect, the disclosure provides the use of a wound healant composition, tissue healant composition, composition, cell composition, platelet concentrate composition, HA composition, chitosan composition, PRP composition, A-PRP composition, thrombin serum, or haemostatic agent obtained by using a hematology tube, syringe, A-PRP kit, PRP kit or medical device according to any of the previous aspects, obtained by using a machine according to any of the previous aspects or obtained by a method according to any of the previous aspects on a wound, a damaged tissue, damaged bone or periodontal defect or cavity.

In another aspect, the disclosure provides a use of a wound healant composition, tissue healant composition, composition, cell composition, platelet concentrate composition, HA composition, chitosan composition, PRP composition, A-PRP composition, thrombin serum, or haemostatic agent obtained by using a hematology tube, syringe, A-PRP kit, PRP kit or medical device according to any of the previous aspects, obtained by using a machine according to any of the previous aspects or obtained by a method according to any of the previous aspects for the manufacture of a medicament for healing of wounds or tissues or for promoting bone or periodontum growth and/or bone and/or tissue regeneration such as skin, cartilage, muscle, tendon, ligament, adipose tissue, cornea, peripheral nerves, spine or bone regeneration.

In another aspect, the disclosure provides the use of a wound healant composition, tissue healant composition, composition, cell composition, platelet concentrate composition, HA composition, chitosan composition, PRP composition, A-PRP composition, thrombin serum, or haemostatic agent by using a hematology tube, syringe, A-PRP kit, PRP kit or medical device according to any of the previous aspects, obtained by using a machine according to any of the previous aspects or obtained by a method according to any of the previous aspects for the manufacture of a cosmetic preparation for use as anti-aging agent or skin repairing agent such as a scar repairing agent, lipoatrophy repairing agent, a wrinkle filling and/or repairing agent, for esthetic preparation, aging management, volume corrector and/or hair stimulator.

In another aspect, the disclosure provides the use of a wound healant composition, tissue healant composition, composition, cell composition, platelet concentrate composition, HA composition, chitosan composition, PRP composition, A-PRP composition, thrombin serum, or haemostatic agent obtained by using a hematology tube, syringe, A-PRP kit, PRP kit or medical device according to any of the previous aspects, obtained by using a machine according to any of the previous aspects or obtained by a method according to any of the previous aspects for the manufacture of a cosmetic preparation for use in dentistry, orthopedics, arthritis, osteoarthritis, pseudo-arthritis or else. In one embodiment, the wound healant composition or tissue healant composition is applied in a dental cavity, on a diabetic ulcer, perforating ulcer, diabetic perforating ulcer, or else.

In one aspect, the wound healant composition, tissue healant composition, composition, cell composition, platelet concentrate composition, HA composition, chitosan composition, PRP composition, A-PRP composition, thrombin serum, or haemostatic agent may be combined with tricalcium phosphate (TCP), with any bone substitute and/or hyaluronic acid/chitosan preferably before the formation of the clot. The composition may be used as volume corrector (TCP at 10-30 microns), in dentistry, orthopedics (TCP at 50 microns).

Combinations include TCP, hyaluronic acid/chitosan and a PRP composition. A preferred combination includes TCP, hyaluronic acid/chitosan and an A-PRP composition. Combinations include TCP, hyaluronic acid, gelose, chitosan, albumin, mannitol, growth factors, ascorbic acid, collagen and/or silk with an A-PRP composition or PRP composition.

The formation of a clot is a multi-step process or cascade and several of these steps require the presence of calcium ions. By removing the calcium ions present in whole blood, as is the effect when the blood is collected in citrate, the blood can be prevented from clotting. A calcium chelating agent (also referred herein as anticoagulant) is a chemical that reacts with the calcium, present in blood, in such a fashion that the calcium can no longer function in blood coagulation. The most common chelating agent is a salt of citric acid (citrate), since it has the fewest side effects on the components of the clotting system. By collecting blood into a medium containing a calcium chelating agent such as citrate, sample collection and further preparations of the citrated sample can be performed over a time period of up to several hours. Preferred calcium chelating agent is sodium citrate.

A buffered sodium citrate solution at about 0.10 M or an anhydrous sodium citrate at about 3.5 mg/mL may be used.

Alternatively, hirudin, benzylsulfonyl-d-Arg-Pro-4-amidinobenzylamide (BAPA), heparin, citrate, acid citrate dextrose (ACD), citrate-theophylline-adenosine-dipyridamole (CTAD) or potassium-ethylenediaminetetra-acid (EDTA) may be used as anticoagulants. Combination of anticoagulants may be used and injected in the hematology tubes via various or same injectors, sequentially or simultaneously.

In one disclosure, instead of thrombin serum, an alternative coagulation activator may be used such as calcium chloride or calcium saccharate, preferably calcium gluconate.

In one disclosure, multiple coagulation activators may be used in combination, preferably thrombin serum with calcium gluconate and optionally calcium saccharate.

Advantageously, the methods of the present disclosure permit manipulation of the blood in an entirely closed circuit during the entire process, from the manufacturing process, blood collection, manipulation till application or injection to the patient. All the devices and kits are therefore adapted for an entirely closed circuit manipulation in order to avoid direct contact of the blood and hematology tubes with air.

Advantageously, the methods of the present disclosure reduce oxidative stress and reduce manipulation time ex-vivo.

Furthermore, the present disclosure provides novel formulations of A-PRP and/or fresh cells comprising hyaluronic acid and/or chitosan and which are suitable for automated manufacturing processes.

In addition to the A-PRP or autologous preparation of fresh cells, there is a need of mechanical and biological support to the cells migration and expansion eventually differentiation. Cells and growth factors need a provisory matrix to enhance their biological expansion.

In one aspect of the disclosure, a composition of the present disclosure is useful as a matrix, semi solid support, cell media or useful for any cell culture. In one aspect, the invention provides a matrix, semi solid support or a cell medium comprising a composition according to the invention. In one embodiment, the matrix, semi solid support or cell media comprises hyaluronic acid and/or chitosan, preferably a HA and/or chitosan composition of the present disclosure. In one embodiment, the matrix, semi solid support or cell media may further comprise a plasma concentrate, preferably PRP. In one disclosure, the matrix, semi solid support or cell media media may further comprise growth factors. In one disclosure, the matrix, semi solid support or cell media media may further comprise at least one cell extract, preferably fresh cell extract or fresh cells. Such cell extract may be any cell extract as herein disclosed, provided the cell extracts are not embryonic stem cell extracts. For example, adipose stem cells, fibroblasts or pancreas cells may be used. In a preferred embodiment, the matrix, semi solid support or cell medium comprising a composition disclosed herein is expanded in vivo.

Advantageously, such cell media is an enriched media favorable for cell expansion and/or cell growth. Such cell media may also represent a suitable media for transport, suitable media of the conservation of cells, tissue or organ. Such media may be useful for grafts and may preserve cells until graft is performed. Once applied on the patient, such matrix, semi solid support or cell medium may be combined with a platelet concentrate or PRP. Such cell media may be suitable for clinical application, as implants, for injection, for application alone or as a bone substitute, but also for research purposes, for example for the development of cell lines.

In one disclosure, the total concentration of the HA and PRP composition, preferably a HA and PRP composition of the present invention, in a cell media is between about 15% to about 60%, preferably 40%. For example, in a HA and PRP cell media, PRP may be present at a concentration of about 5% to about 40%, preferably at a concentration of about 20%, and HA at a concentration from about 10% to about 20%. A concentration higher than 60% of HA and PRP seems inhibitory for cells. Such HA and PRP composition has been tested with adipose stem cells. Advantageously, such composition may replace BSA (Bovine Serum Albumin) or similar substance present in a media. The HA and PRP composition may preferably further comprise a saline solution, DMEM, RPMI and/or any other suitable solution or culture media, preferably at a concentration of about 60%.

Preferably, the disclosure provides a composition comprising fresh cells and growth factors, in a semi solid support of Hyaluronic acid or Chitosan, preferably a HA and/or chitosan composition of the present invention, preferably expanded in vivo, into the patient himself.

The disclosure therefore also provides a new tissue engineering model or composition composed only of fresh cells and growth factors, in a semi solid support of Hyaluronic acid or Chitosan, expanded in vivo, into the patient himself instead of a remote laboratory.

Further uses may include healing of wounds or for promoting bone or periodontum growth and/or bone and/or tissue regeneration.

Further uses may include the manufacture of a cosmetic preparation for use as anti-aging agent or skin repairing agent such as a scar repairing agent, a wrinkle filling and/or repairing agent.

Further uses may include the manufacture of a cosmetic preparation for use as esthetic preparation, aging management, volume corrector, wrinkle feeling, brown spot reduction and/or hair stimulator. Compositions may be applied on and/or around the eyes, lips, eyelids, face, neck, chest, scalp, hair, hands and all the rest of the body and/or male and female genitalia. In one embodiment, the cosmetic preparation and/or esthetic preparation is combined with a cosmetic agent, cosmetic cream or cosmetic mask. Further uses include aesthetics, for example as filler.

The present formulations may be applied on a mask.

Further uses may include ligament and/or cartilage reconstitution. Advantageously, ligament and/or cartilage reconstitution time using a composition of the present invention is divided by a factor 2 or 3 in comparison with known methods.

In another aspect, the present disclosure provides a wound healant composition, tissue healant composition, cell composition, platelet concentrate composition, HA composition, chitosan composition, PRP composition, A-PRP composition, thrombin serum, or haemostatic agent according to the invention and a pharmaceutically or cosmetically acceptable carrier.

Further uses may include the manufacture of a medicament for healing of wounds or tissues, mesotherapy, intramuscular, or for promoting bone or periodontum growth and/or bone and/or tissue regeneration such as skin, cartilage, muscle, tendon, adipose tissue, cornea, peripheral nerves, spine or bone regeneration.

Further uses may include the manufacture of a cosmetic preparation for use as anti-aging agent or skin repairing agent such as scar repairing agent, lipoatrophy repairing agent or wrinkle filling and/or repairing agent.

Further uses may include regeneration and/or rejuvenation of tissues, bones and/or cartilages. Further uses may include the treatment of diabetic neuropathic ulcers or decubitus sores; bone and cartilage damages such as deep joint cartilage or chondral damages such as surgical repair of torn tendons; arthritis in joint caused by traumas or by aging; rotator cuff disorders; non-healing wounds such as non-healing wounds such as vasculitis induced wounds, for example in lower equine limb; periodontal diseases; implant surgery; cardiovascular, thoracic, transplantation, head and neck, oral, gastrointestinal, orthopedic, neurosurgical, and plastic surgery; mesotherapy and/or mesotherapy injections; cardiac muscle damages such as in chronic cardiac failure, heart failure, ischemic and non- ischemic disorders, cardiomyopathy; gastro-oesophageal reflux disease; anal or urinary incontinence; facial surgery such as facial surgery induced alopecia (alopecia due to hair follicle loss in the side burn areas), hair loss, alopecia, face-lift surgery (rhytidectomy), rhinoplasty, dermal fat grafts (in the treatment of facial augmentation, congenital hemiatrophy of the face such as congenital cartilage nose atrophy and lipoatrophy such as in HIV/ AIDS suffering patients, genital dysfunction, erosion and arthroscopy); wound healing complications such as after eyelid blepharoplasty; corneal disorders such as corneal opacity such as those caused by chemical burns, affliction by Steven's Johnson syndrome and corneal ulcers; scarring of the cornea; dry eye syndrome; haematological diseases such as Thalassaemia; peripheral nerve damage, nerve suture and spinal cord injury; bone defects or disorders such as bone graft or bone fracture, skin damages or disorders such as acne (especially after dermabrasion treatment), burns, rubella or small pox scars, vitiligo, lipoatrophy, Kaposi's sarcoma, skin skeloids or Dupuytren's palmar fibromatosis.

Further uses may include tissue healing, including bone regeneration and repair, mitogenesis, angiogenesis and/or macrophage activation.

Additional advantages and novel features of this invention shall be set forth in part in the description that follows, and in part will become apparent to those skilled in the art upon examination of the following specification or may be learned by the practice of the invention. The objects and advantages of the invention may be realized and attained by means pointed out in the appended claims.

Further uses may particularly include haemostasis, the regeneration, revitalization, hydration and/or stimulation of tissue, as biological glue, bioadhesive sealant or biological filler.

Further uses may particularly include wound care, surgery, injections for orthopedic and injections for esthetic, cosmetic or volume corrections.

Further uses may include the regeneration and/or rejuvenation of skin tissues, particularly in promoting and/or initiating skin regeneration such as reducing skin wrinkles, deep wrinkles, acne (especially after dermabrasion treatment), burns, rubella or small pox scars, vitiligo and lipoatrophy (e.g. anti-aging compositions and skin regeneration compositions), amelioration of nasolabial lines and treatment of skin damages or disorders such as skin burns, Kaposi's sarcoma, skin skeloids or Dupuytren's palmar fibromatosis, in the reduction of pain associated with skin and tissue regeneration, for hemorrhoidal cushion, erectile dysfunction, caverna, cavernosal fibrosis, lapeyronie's disease, vagina and/or labia.

Further uses may include wound or tissue healing, regeneration treatments or sports medicine for the knee, elbow, (torn) muscles, spine, spinal disc, tendon, ligament, the treatment of traumatic or surgical wounds such in the fitting and/or holding and/or sealing of native or prosthetic grafts (especially skin, bone grafts and/or dental prostheses or implants or the like, including also the graft donor site); treatment of arthritis, osteoarthritis, gonarthritis, tendinitis, rotator cuff, treatment of vasculitis; ulcers such as diabetic neuropathic ulcers or decubitus sores; radiodermatitis (e.g. after irradiation on an epidermoidal skin carcinoma) and closing fistulas (such as for cyclists).

Further uses may include the treatment of cardiac disorders, cardiac regeneration such as in the treatment of heart failure, chronic cardiac failure, ischemic and non-ischemic cardiac failure and cardiomyopathy.

Further uses may include the treatment of urinary and/or anal incontinence.

Further uses may include the treatment of reflux oesophagitis and/or gastro-oesophageal reflux disorder.

Further uses may include the treatment of skin damages such as in skins damaged by radiations (radiodermatitis or sun damaged skin), aged skins or burned skins and/or in the amelioration of facial wrinkles, rhytids, acne (especially after dermabrasion treatment), burns, rubella or small pox scars, vitiligo, lipoatrophy or lipodystrophy, Kaposi's sarcoma, skin skeloids or Dupuytren's palmar fibromatosis and/or in skin rejuvenation treatments.

Further uses may include the treatment of lipoatrophy such as in HIV/AIDS patients and in other congenital hemiatrophy of the face such as congenital cartilage nose atrophy. Further uses may include the treatment of bone, cartilage and articular disorders such as chondral damage, arthritis, osteoarthritis, cartilage and/or bone injury such as deep cartilage damage and/or erosion and/or arthroscopy, tendon torn and rotator cuff in shoulder.

Further uses may include the treatment of hematological diseases such as Thalassaemia.

Further uses may include the treatment of corneal disorders such as dry eye syndrome; corneal opacity such as those caused by chemical burns, affliction by Steven's Johnson syndrome; scarring of the cornea and corneal ulcers.

Further uses may particularly include the treatment of peripheral nerve damage, Schwann cell damage, glial cell damage, neurons damage, nerve suture and spinal cord injury.

Further uses may particularly include the treatment of type I diabetes, insulin-dependent diabetes and/or hyperglycaemia.

Further uses may include the treatment of bone defects or disorders such as bone graft or bone fracture.

The use of the resulting composition of the invention can be further modified before application and according to the therapeutic objective.

Compositions of the invention can be used together with bone filling materials, especially resorbable filling materials such as hydroxyapatite (calcium phosphate ceramic used as a biomaterial) or demineralised bone, or used as a mixture with bone extracts in a process for the regrowth of bone for example in craniofacial and orthopaedic procedures.

Further uses may include orthopaedics for example as visco-supplementation or for bone reconstruction using a combination of the present formulations with stem cells, cell extract and/or TCP, provided the cells or cell extracts are not embryonic stem cells or cell extracts.

Further uses may include as wound sealant in plastic surgery including burn grafting and other free skin graft applications, for example in oncology for favouring tissue regeneration, including speeding (neo)vascularization. Further uses may include in wound healing treatments at the skin graft donor site.

Further uses may include wound care as biological glue, for example for burns or diabetic ulcers.

Further uses may particularly include the treatment of chronic wounds that may lack sufficient blood circulation to facilitate the wound healing cascade.

Further uses may include the treatment of periodontal disease where a loss and/or a damage of the periodontal tissues is observed, such a treatment comprising for example placing at the periodontal site or cavity in a human or a lower animal in need of periodontal tissue regeneration a composition according to the disclosure . Further uses may include eliminating or greatly reducing post-operative bleeding and extravasation or loss of serous or other fluid in these applications, reducing the infection risk caused by most bacteria and/or enhances connective tissue formation compared to natural healing (i.e. no exogenous agents added) or improve healing obtained through the use of other platelet concentrates, PRP compositions prepared with known methods.

Further uses may particularly include promoting and/or initiating wound healing and/or tissue regeneration or for the preparation of cosmetic compositions for skin regeneration such as reducing skin wrinkles, acne (especially after dermabrasion treatment), rubella or small pox scars, vitiligo and lipoatrophy (e.g. anti-aging compositions and skin regeneration compositions).

The compositions obtained by the manufacturing processes of the present disclosure may be administered locally or injected in the wound or in or near to the grafted organ or injected subcutaneously. Local administration may be by injection at the site of injury or defect or by insertion or attachment of a solid carrier at the site, or by admixture with a cream or emulsion, or by inclusion in a tissue or paper or hydrogel carrier, or by direct, topical application of the composition of the invention such as in the form of eye drops. Preferably, the compositions are readily syringable compositions. The mode of administration, the dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

The compositions of the disclosure or the compositions obtained by the manufacturing processes of the present disclosure may be administered in combination with a co-agent useful in the treatment of tissue regeneration such as a healing agent, a wrinkle filler, an anti-aging agent such as an anti-aging vitamin complex, an antibacterial agent, antibiotic agent, an corticosteroid agent, an antalgic and analgesic agent, or an anesthetic agent like adrenaline, etc... The compositions obtained by the manufacturing processes of the present disclosure may be combined with a co-agent useful in the treatment of tissue regeneration for simultaneous, separate or sequential use in tissue regeneration therapy such as wound healing, bone and periodontum growth repair.

Further uses may include therapeutic use, particularly as autogenous biological glue in a haemostatic system intended to accelerate the physiological process of tissue regeneration, for example in dental implantology, skin and bone surgery, cartilage and tendon surgery, orthopedics, corneal and peripheral nerve regeneration and cardiac surgery. Further uses may particularly include cosmetic use, particularly as autogenous rejuvenation material intended to be used for example as wrinkle, scar or fat deficiency filler, alone on in combination with at least one anti- aging agent. Further uses may particularly include the acceleration and/or promotion of the healing process of wounds, even chronic unhealing wounds, leading to successful closures where weeks of conventional therapies had failed and achieving a decrease in infection risks, an improvement in patient's recover and comfort, a reduction of medical care costs and a better esthetic final result.

The compositions, hematology tubes or syringes may also use plasma derived from several identified donors. The disclosure is not limited to autologous biological materials, such as collection of concentrated platelets from the wounded own biological material. The disclosure encompasses the use of biological materials obtained from one or more third parties, who need not be of the same species as the patient whose wound is being treated with the compositions described herein unless bio-incompatibility would result from the use of such third party biological materials.

Further uses may include sealing a surgical wound by applying to the wound a suitable amount platelet concentrate, PRP composition, A-PRP composition once it has begun to gel. Moreover, due to the high quality standards in place for the manufacturing process and to the fact that the wound or tissue healant compositions may be prepared solely from blood components derived from the patient that is to receive the wound or tissue healant compositions there is a zero probability of introducing a new blood transmitted disease to the patient.

A wide variety of drugs or proteins with other biologic activities may be added to the compositions herein described. Examples of the agents to be added to the compositions (for example prior to the addition of the serum) include, but are not limited to, analgesic compounds, antibacterial compounds, including bactericidal and bacteriostatic compounds, antibiotics (e.g., adriamycin, erythromycin, gentimycin, penicillin, tobramycin), antifungal compounds, anti-inflammatories, antiparasitic compounds, antiviral compounds, enzymes, enzyme inhibitors, glycoproteins, growth factors, recombined (e.g. lymphokines, cytokines), hormones, steroids, glucocorticosteroids, immunomodulators, immunoglobulins, minerals, neuroleptics, proteins, peptides, lipoproteins, tumoricidal compounds, tumorstatic compounds, toxins and vitamins (e.g., Vitamin A, Vitamin E, Vitamin B, Vitamin C, Vitamin D, or derivatives thereof).

In one embodiment, compositions herein described can be admixed with a coagulation activator, thrombin serum, tricalcium phosphate (TCP), a bone substitute, hyaluronic acid composition, calcium gluconate, calcium saccharate, chitosan, fibroins, silk-fibroin proteins, growth factors, mannitol, collagen, albumin, ascorbic acid, cream, fat cells, fat tissue, bone marrow concentrate, lubricin, cd-gelatin, botulinum toxin and/or one or more cell extracts, which are not embryonic stem cell extracts. Such substances may be combined during the preparation of the hematology tubes or syringes (via for example an injector) or after the preparation of the hematology tubes or syringes as herein described.

**Examples**

Example 1

Different hyaluronic acids have been tested for sterilization according to table 1.

Table 1

| Nom | Lot | Intrinsic Viscosity (m3/Kg) | Average Molecular Weight (KDa) |
|---|---|---|---|
| **HA1** | H1909S01 | 1.08 | 720 |
| **HA2** | H1289S01 | 1.91 | 1550 |
| **HA3** | H2046 | 2.52 | 2480 |
| **HA4** | H2143 | 3.19 | 3480 |
| **HA 500 KDa** | PHI2168S02 | 0.81 | 500 |
| **HA 4100KDa** | PHI2374 | 3.57 | 4100 |

The different HA solutions HA1, HA2, HA3 and HA4 have been tested in normal conditions (control), after steam sterilization (8 minutes at 105°c) or Gamma irradiation (normal cycle for production).
Textures and appearance of the different original solutions HA1, HA2, HA3 and HA4 are shown in table 2.

Table 2

| | | Texture / appearance | | |
|---|---|---|---|---|
| | | 2% | 3% | 4% |
| | HA1 | Liquid (gelified), colorless | Liquid (gelified), colorless | Liquid (gelified), colorless |
| | HA2 | Liquid (gelified), colorless | Liquid (gelified), colorless | Liquid (gelified), colorless |
| | HA3 | Liquid (gelified), colorless | Liquid (gelified), colorless | Liquid (gelified), colorless |
| | HA4 | Liquid (gelified), colorless | Liquid (gelified), colorless | Liquid (gelified), colorless |

The solutions are considered 'solid' when they do not flow and are able to maintain they original form. The gel state refers to a particular persistent viscosity.
The different HA solutions HA1, HA2, HA3 and HA4 after gamma irradiation are shown in table 3.

Table 3

| | Texture / appearance | | |
|---|---|---|---|
| | 2% | 3% | 4% |
| HA1 | Liquid, brown | Liquid, brown | Liquid, brown |
| HA2 | Liquid, brown | Liquid, brown | Liquid, brown |
| HA3 | Liquid, brown | Liquid, brown | Liquid, brown |

(continued)

| | Texture / appearance | | |
|---|---|---|---|
| | 2% | 3% | 4% |
| HA4 | Liquid, brown | Liquid, brown | Liquid, brown |

The brown color of all the different HA solutions mean a probable denaturation or alteration of HA following gamma irradiation.

Solution HA2 has been tested in various concentrations after steam sterilization as shown in table 4.

Table 4

| | Texture / appearance | | |
|---|---|---|---|
| | 2% | 3% | 4% |
| HA2 | Gelified, colorless | Gelified, colorless | Gelified, colorless |

Sterilization by gamma radiation is inadequate. Only steam sterilization permits conservation of the original HA appearance and maintenance of HA function, for example in visco supplementation.

**Example 2**

Viscosity measures have been performed on different solutions of hyaluronic acid and mixture of hyaluronic acid and PRP.

Measured viscosity of the different HA solutions HA1, HA2, HA3, HA4 are shown in table 5.

Table 5

|  | Average initial viscosity (mPa.S) | Viscosity following gamma irradiation (mPa.S) | Viscosity following steam sterilization (8' à 105°c) (mPa.S) |
|---|---|---|---|
| HA1 - 2% | 2700 | 300 | N/A |
| HA1 - 3% | 8400 | 0 | N/A |
| HA1 - 4% | 18700 | 300 | N/A |
| HA2 - 2% | 8070 | 100 | 6280 |
| HA2 - 3% | 19000 | 100 | (*) (8' à 105°c) |
| HA2 - 4% | 22250 | 0 | (*) (8' à 105°c) |
| HA3 - 2% | 9800 | 0 | N/A |
| HA3 - 3% | 15200 | 0 | N/A |
| HA3 - 4% | 31200 | 0 | N/A |
| HA4 - 2% | 9100 | 0 | N/A |
| HA4 - 3% | 20300 | 0 | N/A |
| HA4 - 4% | 31000 | 100 | N/A |
| 500KDa - 1% | 0 | - | - |
| 500KDa - 2% | 300 | - | - |
| 500KDa - 3% | 5700 | - | - |
| 500KDa - 4% | 10100 | - | - |
| 4000KDa - 1% | 3200 | - | - |
| 4000KDa - 2% | 11440 | - | 10340 |
| 4000KDa - 3% | 22000 | - | - |

(*) Non-measurable value with viscometer because too high value.

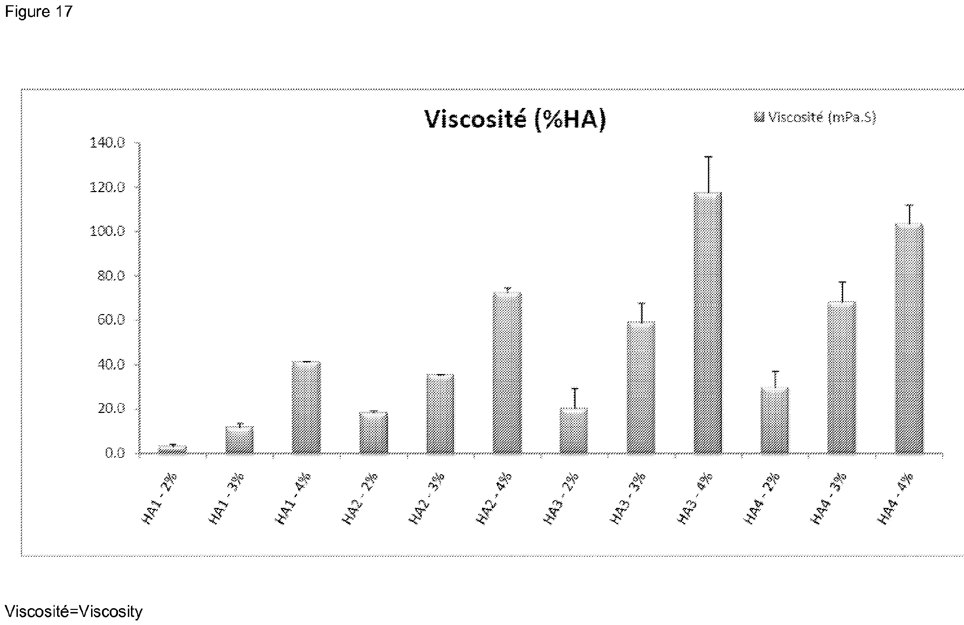

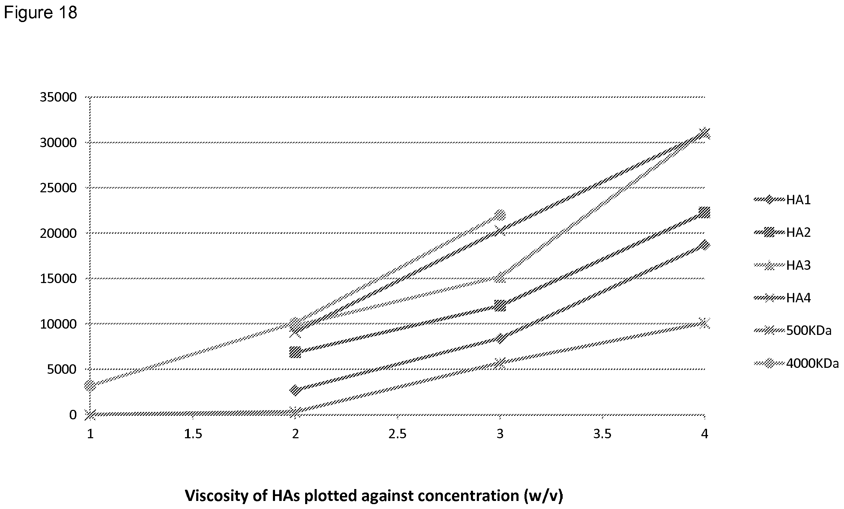

Viscosity has been measured for various HAs (see Figure 17). Viscosity increases with molecular weight and/or HA concentration, see also Figure 18.

Sterilization by gamma radiation reduces by 95%-100% HA's viscosity, confirming denaturation and/or cleaved links implicated in viscosity.

Sterilization by steam enables conservation or even improvement of HA solution's viscosity.

Viscosity measures on mixtures of hyaluronic acid and PRP have been made on a total of 18 patients is shown in table 7. HA2 was tested at a concentration of 2%. Tubes have been steam sterilized before use. 4ml of PRP-HA mixture has been tested, in equal quantity. Control is made on a mixture of HA and physiologic serum in equal quantity and is shown in table 6.

Table 6

| Control | (mPa.S) |
|---|---|
| 1 | 400 |
| 2 | 300 |
| 3 | 300 |

(continued)

| Control | (mPa.S) |
|---------|---------|
| Average | 333 |
| SEM | 44 |

Table 7

| Patient | Viscosity (mPa.S) | Viscosity (mPa.S) |
|---------|-------------------|-------------------|
| Patient 6 | 200 | 250 |
| Patient 7 | 400 | 500 |
| Patient 8 | 400 | 500 |
| Patient 9 | 350 | 500 |
| Patient 10 | 200 | 300 |
| Patient 11 | 200 | 400 |
| Patient 12 | 400 | 600 |
| Patient 13 | 500 | 350 |
| Patient 14 | 200 | 250 |
| Patient 15 | 400 | 450 |
| Patient 16 | 500 | 550 |
| Patient 17 | 400 | 300 |
| Patient 18 | 300 | 300 |
| Patient 19 | 600 | 450 |
| Patient 20 | 300 | 300 |
| Patient 21 | 200 | 450 |
| Patient 22 | 200 | 400 |
| Patient 23 | 200 | 300 |
| Mean | 331 | 397 |
| SEM | 108 | 92 |

Table 8 shows the different solutions tested, mixtures of HA differing in molecular weight and concentration (solutions F to I).

Table 8

| | 500KDa (%) | 4000KDa (%) |
|---|------------|-------------|
| F | 1 | 1.5 |
| G | 1.5 | 1.5 |
| H | 2 | 1.5 |
| I | 2 | 1.25 |

Viscosities of the PRP-HA mixtures (F to I) are comparable to the controls Ostenil (see Table 9). Viscosity remains stable *in vitro,* indicating a good stability of the mixture at room temperature. PRP having viscosity similar to water (0 mPa.s), addition of HA is a reliable mean in order to increase PRP's viscosity.

Table 9

| | Texture | Viscosity (mPa.S) | Viscosity after Steam (mPa.S) |
|---|---|---|---|
| F | gelified | 12700 | 11133 |
| G | gelified | 13200 | - |
| H | gelified | 14200 | - |
| I | Gelified, less viscous than H | 9400 | - |
| Ostenil 1%* | Liquid, not runny, non gluey | - | 2000 |
| Ostenil 2%* | Gelified, not runny, sparsely gluey | - | 12100 |

Flow with syringe and needgle 25G was tested for the various solutions (Table 10). The solutions F and I are able to flow and are suitable for industrial application.

Table 10

| | Flow |
|---|---|
| F | + |
| G | - |
| H | - |
| I | + |
| Ostenil 1% | ++++ |
| Ostenil 2% | ++ |

The texture of solution F guarantees the best viscosity with good manipulability. Advantageously, it procures a higher concentration of HA (about 2.5%) while ensuring a slow resorption. Solution I is also acceptable, providing a higher concentration of HA of about 3.25%.

Advantageously, the HA formulations of the present invention do not require presence of an adjuvant like ostenil. Mannitol is added to ostenil 2% in order to stabilize the hyaluronic acid. Ostenil is a single molecular weight HA. The presence of a high molecular weight HA in the solutions of the present invention procures stability. Moreover, advantageously, the high molecular weight HA has a long-lasting effect because it is metabolized more slowly than a HA of lower molecular weight like ostenil.

In contrast, the presence of a low molecular weight HA in the solutions of the present invention may favor cellular growth. Advantageously, it will be metabolized more rapidly, with a faster action on the visco supplementation, because such a low molecular weight HA is more penetrating than an HA of higher molecular weight like ostenil.

**Example 3**

Material and Methods

*A) STUDY DESING*

The whole blood collected from 23 donors was processed using two devices in parallel called RegenBCT tube for PRP alone preparation and RegenBCT-HA tube for a preparation combining PRP and hyaluronic acid. In order to establish the performances regarding blood component separation, the following measures were carried out using the whole blood and corresponding PRP at time 0 (T=0) and 4 hours after blood collection (T=4H):

- pH values
- Counts of blood cells and platelets
- Evaluation of platelet and cell recovery, and platelet concentration factor

Moreover, in order to establish platelet integrity and functionality, the following experiments were carried out using the PRP obtained with the two devices at times 0 and 4 hours after blood collection:

- P-selectin expression on resting and ADP activated platelets
- Hypotonic stress response of the platelets
- Platelet aggregation in presence of collagen

BCT herein stands for Blood Cell Therapy(ies) or Blood Collection Tube(s).

*B) PLATELET SEPARATION SYSTEMS*

*RegenBCT tube*

The RegenBCT tube is a vacuum tube in which 8 to 9 ml of blood are automatically collected. It uses a thixotropic gel for cell separation and sodium citrate as anticoagulant agent. After centrifugation, blood components are separated. Red blood cells are sequestered below the gel while platelets and plasma are over the gel. During the centrifugation, platelets and the remaining blood cells have settled on the upper surface of the gel therefore they must be put back in suspension in the plasma before collecting it. This is done by gentle inversion of the tubes. Platelet rich plasma can then easily be collected from the tube directly in the syringe that will be use for injection.

*RegenBCT-HA tube*

The RegenBCT-HA tube is a variant of the RegenBCT tube. It uses the same gel technology to produce PRP, however from around 5 ml of blood. In addition this tube also contains hyaluronic acid that will be combined with the PRP. Before blood withdrawal, the hyaluronic acid is at the bottom of the tube, covered by the separating gel and the anticoagulant. During the centrifugation the red blood cells will migrate at the bottom of the tube while the hyaluronic acid will move in the upper part or the tube. At the end of the centrifugation, red blood cells are sequestered below the gel, the plasma with the platelets are over the gel, covered by the layer of hyaluronic acid. As in RegenBCT tubes, cellular elements and platelets will be settled on the surface of the gel and must be put back in suspension by gentle agitation of the tube. The hyaluronic acid is then mixed with the PRP and collected with the syringe that will be use for injection.

*C) BLOOD COLLECTION*

Blood was collected from 23 volunteers over a period of two weeks. Each donor was informed of the procedure and gave a written informed consent.

Around 24 ml of blood were withdrawn from the medial cubital vein of each donor using a BD Vacutainer®Safety-Lok™ Blood Collection Set connected to a BD collection holder (Becton Dickinson AG, Switzerland) in which the vacuum tubes are inserted for automatic blood collection. For each donor, blood was taken in one RegenBCT tube and three RegenBCT-HA tubes for the PRP and PRP+HA preparations, and four ml of blood were collected in a small BD Vacutainer® citrate tube (Becton Dickinson AG, Switzerland) for whole blood values determination.

Blood volume in each device was determined by weighing.

*D) ANALYSIS OF PLATELET-RICH PLASMA (PRP) PREPARATION*

*PRP preparation*

PRPs were obtained by centrifugation of the two types of devices according to their respective protocol. Tubes were centrifuged, using a RegenPRP Centri (Regen Lab SA; Switzerland), 5 min. at 3400 rpm, speed corresponding to a relative centrifugal force of 1500 g. All centrifugations were performed at room temperature ($\approx$ 22°C).

After centrifugation, the RegenBCT tube was gently agitated by inversion 5 to 10 times to re- suspend the platelets and cells, which had settled on the upper surface of the separating gel, in the plasma. The volume of PRP obtained was measured using a 5 ml-Luer-Lok™ syringe connected to an 18G BD Blunt Fill Needle.

In the RegenBCT-HA tubes, blood components were separated by the separating gel like in the RegenBCT tube, but the plasma was covered by the layer of hyaluronic acid.

Unfortunately, due to the viscosity of the product, the mix of PRP with hyaluronic acid can't be used on automated hematological cell counter and for the testing of platelet aggregation. To circumvent this problem, blood was withdrawn in three RegenBCT-HA tubes for each donor. One tube was used to prepare the mix of PRP with hyaluronic acid, while the two other tubes were used to evaluate the PRP alone, one for time T=0 and the other for the measures after four hours.

In the first RegenBCT-HA tube (group A), platelets and cells, which had settled on the surface of the separating gel, were resuspended in the plasma by gentle inversion. The resulting PRP was then mixed with the hyaluronic acid and volume of the PRP-HA mix was measured using a 5 ml-Luer-Lok™ syringe connected to an 18G BD Blunt Fill Needle. The PRP-HA mix was used for pH measure and the functionality and integrity of the platelets in the mix were tested by their ability to express P selectin in response to ADP stimulation and to resist to a hypotonic stress.

In the second RegenBCT-HA tube (group B), directly after the centrifugation step, the upper layer of hyaluronic acid was gently removed by aspiration in a 5 ml-Luer-Lok™ syringe connected to an 18G BD Blunt Fill Needle. While doing this some of the upper part of the plasma, was also collected. This plasma was considered free of cellular elements, as platelets and cells were still sedimented at the surface of the separating gel. The volume of hyaluronic acid and plasma withdrawn was evaluated by weighting. The platelets and cells were then resuspended in the remaining plasma by gentle inversion of the tube. The volume of PRP obtained was measured using a 5 ml-Luer-Lok™ syringe connected to an 18G BD Blunt Fill Needle. This PRP was used for evaluation of platelet and cell counts and platelet aggregation in response to collagen at time T=0.

The third RegenBCT-HA tube (group C) was left untouched after the centrifugation in vertical position for four hours. At time T=4H the upper layer of hyaluronic acid was gently removed by aspiration, following the same procedure that was done at time T=0 for the RegenBCT-HA tube of group B. The PRP obtained was used for evaluation of platelet and cell counts and platelet aggregation in response to collagen at time T=4H.

As the hyaluronic acid and cell free plasma volume that was removed was evaluated for each RegenBCT-HA tubes of groups B and C, we were able to estimate by calculation the final concentration of the different blood elements in those tubes.

*pH determination*

The pH of whole blood, PRP and PRP-HA mix preparations were measured using a MP 230 pH-meter connected to an Inlab® Semi-Micro electrode (Mettler Toledo, Switzerland). The pH-meter was calibrated daily using Fluka standardized buffered solutions pH 7 and pH 4 (Sigma Aldrich Chemie, Switzerland).

*Counts of blood cells and platelets*

Count of blood cells and platelets in whole blood and PRP preparations were carried out using a Micros 60 automated hematology analyzer (Horiba ABX, Montpellier, France. Quality control tests of the analyzer were performed each day of measurements with control blood (Minotrol 16-2N, Horiba ABX, Montpellier, France).

*Platelet and cell recovery*

The percentages of platelet and cell recovery, obtained using the RegenBCT and RegenBCT-HA tubes, as well as the platelet concentration factors, were determined using the following formulas:

$$\textbf{Recovery (\%)} = \frac{(\text{PRP volume}) \times (\text{concentration in PRP}) \times 100}{(\text{whole blood volume}) \times (\text{concentration in whole blood})}$$

$$\textbf{Concentration factor} = \frac{(\text{concentration in PRP})}{(\text{concentration in whole blood})}$$

*E) EVALUATION OF PLATELET FUNCTIONALITY*

In order to verify their integrity and functionality, platelets obtained with RegenBCT and RegenBCT-HA tubes and were analyzed for P-selectin expression, hypotonic stress response and aggregation at time 0 and 4 hours after blood collection.

*Evaluation of P-selectin by direct Immunofluorescence staining*

Two platelet markers were quantified, CD62P (P-selectin) for activated platelets and CD61 (Integrin β3), which is an activation-independent platelet marker.

PRP-HA mixes were diluted four times, and PRP to a concentration of $40*10^6$ platelets / ml, with a physiological saline solution. Ten μl of diluted platelet suspension were activated with 10 μl of Adenosine Diphosphate (ADP) (Applichem, AxonLab, Switzerland) to a final concentration of 20 μM, while 10 μl of Dubelcco's phosphate buffered saline (PBS) (Gibco, Invitrogen, UK) were added in controls. The different aliquots of platelet suspensions were incubated with 15 μl of FITC anti-human CD61 (BioLegend, USA) and 15 μl of PE anti-human CD62P (BioLegend, USA) for 20 min. at room temperature. The reactions were blocked and platelets were fixed by adding 450 μl of a cold (4°C) solution of 1% formaldehyde (Fluka, Sigma Aldrich Chemie, Switzerland) in PBS.

The stained PRP preparations were analyzed for their immunofluorescence content using a C6 Flow Cytometer® (Accuri Cytometers Ltd., UK) available at the Flow Cytometry Core Facility of the Ecole Polytechnique Fédérale de Lausanne, Switzerland. Raw data were analyzed using the CFlow® software (Accuri Cytometers Ltd., UK). Quality control of the instrument was made daily by the head of the core facility. The platelet population was identified by its light scatter characteristics and its identity was confirmed with the CD61 staining. CD62P expression on platelets was measured on the gated CD61 positive population.

*Hypotonic stress response*

The platelet response to hypotonic stress was evaluated using the method described by Farrugia et al (J Clin Pathol 1989; 42:1298-1301). 450 μl of the different platelet suspensions, previously diluted with a physiological saline solution to a concentration of $40*10^6$ platelets/ml for RegenBCT PRP and four time for the PRP-HA mix, were placed in a single-use micro cell in the spectrophotometer's cell holder of a Photometer 4010 (Clinicon, Germany). The hypotonic stress was induced by adding 450 μl of distilled water and the variation in light absorbance at 405 nm was monitored during a period of 5 min. The difference between the absorbance readings at 5 min and at 30 sec (minimum absorbance) was defined as the hypotonic stress response (HSR) of the platelet preparation.

*Platelet aggregation*

Platelet aggregation in response to collagen was by turbidimetric measurement of cell suspension in an APACT 4 aggregometer (LABiTec LAbor BioMedical Technologies GmbH, Germany). This test could not be done on PRP-HA mixes due to the viscosity of the preparations therefore this test was done only on PRP preparations.

Platelet-rich plasma is not very pervious for long-wavelength light but platelet free plasma on the other hand is more permeable. The increase of light transmitting capacity of the plasma, occurring during platelet aggregation, can be used for aggregation quantification. Measures are done at 37°C in micro-cuvettes (Cuvettes Dispo System, Endotell AG, Switzerland) containing a small magnetic stirrer for constant agitation of the preparation. To adjust the transmission differences, which occur for various platelet-rich plasmas, PRP is set to a value of 0% transmission and platelet poor plasma (PPP), which occurs after complete aggregation, to a value of 100% transmission.

200 μl of PPP for each sample, were obtained by centrifugation at 1500g for 3 minutes of 250 μl of the corresponding PRP and collecting the supernatant free of platelets.

Platelet aggregation was induced in PRP by addition of collagen (Kollagenreagens Horm, Nycomed Austria GmbH, Germany) at a final concentration of 10 μg / ml. Modification of turbidimetry was recorded during 5 min and the measure at the end of this period corresponded to the percentage of aggregation.

*F) STATISTICAL ANALYSIS*

For all parameters tested, measured data were expressed as the mean ± standard deviation (SD). Statistical analysis was performed using GraphPad InStat version 3.10 (GraphPad Software, USA). Repeated Measures Analysis of Variance and Bonferroni Multiple Comparisons Test (paired data) were applied to analyze whether values obtained in blood and with the two devices were statistically different or not and if they were stable during four hours. Paired T tests were used when only two groups of data were compared.

Results

*A. ANALYSIS OF PLATELET-RICH PLASMA PREPARATIONS*

*1. Donors' data*

Blood samples were collected from 23 healthy volunteers, 15 men (M) and 8 women (F), aged from 23 to 62 years. The mean age was $40 \pm 11$ years.

*2. Blood and PRP volumes*

See Figure 4

Table 11

| | Blood volume | PRP volume | Removed volume | PRP-HA-MIX |
|---|---|---|---|---|
| RegenBCT PRP | $9.38 \pm 0.54$ | $5.13 \pm 0.30$ | | |
| RegenBCT-HA A | $5.52 \pm 0.22$ | | | $5.19 \pm 0.31$ |
| RegenBCT-HA B | $5.51 \pm 0.21$ | $2.45 \pm 0.44$ | $2.69 \pm 0.33$ | $5.14 \pm 0.38$ |
| RegenBCT-HA C | $5.47 \pm 0.22$ | $2.43 \pm 0.47$ | $2.78 \pm 0.38$ | $5.21 \pm 0.29$ |
| p value between BCT-HA A, B & C | ns p >0.05 | ns p >0.05 | ns p >0.05 | ns p >0.05 |
| Mean values obtained from 23 donors; (ns: non-significant) | | | | |

In RegenBCT tubes the mean volume of PRP obtained was $5.13 \pm 0.30$ ml from $9.38 \pm 0.54$ ml of blood.
The three series of RegenBCT-HA tubes gave similar results ($p > 0.05$) regarding blood collection (around 5.5 ml) and volume of PRP-HA MIX obtained (around 5.2 ml).
The mean volume of PRP obtained in RegenBCT-HA tubes of group B at time T=0 was similar to the one collected in group C at time T=4H. Accordingly the mean volume of hyaluronic acid and plasma removed in the two series of tubes was also equivalent. The calculated final volumes were equal to the final volume of PRP-HA MIX obtained in the A group.

*3. pH values*

See Figure 5

Table 12

| | pH at T=0 | pH at T=4H | p value T=0 vs T=4H |
|---|---|---|---|
| Whole blood | $7.43 \pm 0.09$ | $7.38 \pm 0.08$ | ns p >0.05 |
| RegenBCT PRP | $7.67 \pm 0.06$ | $7.79 \pm 0.07$ | ***p<0.001 |
| RegenBCT-HA MIX | $7.59 \pm 0.09$ | $7.67 \pm 0.06$ | **p<0.01 |
| P value WB vs PRP | ***p<0.001 | ***p<0.001 | |
| P value WB vs HA MIX | ***p<0.001 | ***p<0.001 | |
| P value PRP vs PRP HA MIX | **p<0.01 | ***p<0.001 | |
| Mean values obtained from 23 donors; (WB: whole blood; ns: non-significant; **: very significant; ***: highly significant) | | | |

The pH was evaluated in whole blood, in PRP obtained with RegenBCT tubes and in PRP-HA MIX obtained with the RegenBCT-HA tubes of group A.
All pH values were over the FDA requested standard limit of 6.2. The pHs of PRP and of PRP-HA MIX were slightly higher than the blood pH, the highest values were observed in the PRP.
A small increase of the pH value of the two PRP preparations was observed during the 4 hours of the experiment, contrary to the pH of the whole blood which did not vary.

*4. Concentration of red blood cells (RBC) in whole blood and in PRP preparations*

See Figure 6.

Table 13

| | RBC $10^6/mm^3$ at T=0 | RBC $10^6/mm^3$ at T=4H | p value T=0 vs T=4H |
|---|---|---|---|
| Whole blood | 4.51 ± 0.34 | 4.48 ± 0.35 | ns p>0.05 |
| RegenBCT PRP | 0.04 ± 0.03 | 0.04 ± 0.03 | ns p>0.05 |
| RegenBCT-HA PRP | 0.06 ± 0.03 | 0.07 ± 0.14 | ns p>0.05 |
| RegenBCT-HA MIX | 0.03 ± 0.01 | 0.03 ± 0.03 | ns p>0.05 |
| p value PRP vs PRP-HA | ns p>0.05 | ns p>0.05 | |
| Mean values obtained from 23 donors; (ns: non-significant) | | | |

As mentioned before, cells and platelets in PRP-HA MIX couldn't be counted with an automated hematological cell counter. Therefore all the cell and platelet numerations were performed only on blood and PRP preparations. For RegenBCT-HA, numerations at time T=0 were done on PRP obtained after hyaluronic acid removal in RegenBCT-HA tubes from group B, while results at time T=4H were measured in PRP from RegenBCT-HA tubes from group C, where hyaluronic acid was removed only after four hours.

The RegenBCT-HA tubes presented equivalent performance ($p > 0.05$) in removing red blood cells (RBC recovery in PRP-HA mix: 0.49 ± 0.25 %) as the RegenBCT tube (RBC recovery in PRP: 0.47 ± 0.30%). Concentrations of red blood cells were stable during the 4 hours of the test ($p > 0.05$) in blood and in RegenBCT PRP. There was no difference between the PRP preparations isolated from RegenBCT-HA tubes from group B at time t=0 and from Regen-BCT-HA tubes from group C at time t=4H ($p > 0.05$).

The red blood cells being the most dense blood components, if some of them are still in the plasma at the end of the centrifugation, they will be at the upper surface of the separating gel.

Therefore, we can assume that the amount of red blood cells was negligible in the removed volume of hyaluronic acid and plasma.

The calculated final concentration of red blood cells in the PRP-HA mixes was $0.03.10^6/mm^3$.

*5. Concentration of white blood cells (WBC) in whole blood and in PRP preparations*

See Figure 7

Table 14

| | WBC $10^3/mm^3$ at T=0 | WBC $10^3/mm^3$ at T=4H | p value T=0 vs T=4H |
|---|---|---|---|
| Whole blood | 6.18 ± 1.89 | 6.09 ± 1.81 | ns p>0.05 |
| RegenBCT PRP | 1.59 ± 0.53 | 2.02 ± 0.71 | ns p>0.05 |
| RegenBCT-HA PRP | 2.50 ± 1.52 | 2.23 ± 1.82 | ns p>0.05 |
| RegenBCT-HA MIX | 1.18 ± 0.72 | 0.99 ± 0.77 | ns p>0.05 |
| p value PRP vs PRP-HA | ns p>0.05 | ns p>0.05 | |
| Mean values obtained from 23 donors; (ns: non-significant) | | | |

Both type of tubes allowed a reduction of the white blood cells concentration in PRP when compared to whole blood (WB) ($p < 0.001$, not shown in the table).

The differences between the two kinds of PRP preparations were considered statistically not significant ($p > 0.05$). There was no variation observed for the concentration of the WBC, in the whole blood and in PRP preparations, during the period of 4 hours ($p > 0.05$ for all).

As for contaminating red blood cells, the WBC collected in the plasma are on the upper surface of the separating gel at the end of the centrifugation, therefore we think unlikely that a significant amount of them were lost when the layer of hyaluronic acid was removed.

The estimated final concentration of white blood cells in the PRP-HA mixes was around $1.10^3/mm^3$.

*6. Recovery of white blood cells (WBC) in PRP preparations*

See Figure 8.

Table 15

|  | % WBC recovery at T=0 | % WBC recovery at T=4H | p value T=0 vs T=4H |
|---|---|---|---|
| RegenBCT PRP | 13.63 $\pm$ 5.28 % | 17.09 $\pm$ 6.10 % | ns p>0.05 |
| RegenBCT-HA PRP | 17.06 $\pm$ 12.44 % | 14.56 $\pm$ 10.66 % | ns p>0.05 |
| p value PRP vs PRP-HA | ns p>0.05 | ns p>0.05 | |
| Mean values obtained from 23 donors; (ns: non-significant) | | | |

There was no difference between the two kinds of PRP preparations in the percentage of WBC recovered and the values were stable in time (p > 0.05).
These similar results supported our hypothesis that the amount of white blood cells that could have been removed with the hyaluronic acid in RegenBCT-HA was insignificant.

*7. Repartition of white blood cells (WBC) in whole blood and in PRP preparations*

See Figure 9.

Table 16

|  | Granulocytes at T=0 | Granulocytes at T=4H | Mononuclear cells at T=0 | Mononuclear cells at T=4H |
|---|---|---|---|---|
| Whole blood | 65% | 64% | 35% | 34% |
| RegenBCT PRP | 21% | 22% | 79% | 78% |
| RegenBCT-HA PRP | 18% | 23% | 82% | 77% |
| Mean values obtained from 23 donors | | | | |

The repartition in white blood cell subsets was evaluated in blood and in PRP preparations.
In whole blood most of the white blood cells (65%) are granulocytes. The remaining cells (35%) are the mononuclear cells, which are the lymphocytes and the monocytes.
The separating gel in RegenBCT and RegenBCT-HA tubes allows a reduction of the white blood cells in the PRP with the preferential removal of granulocytes. This results in a different repartition of the white blood in RegenBCT PRP and RegenBCT-HA PRP, with around 20% of granulocytes and 80% of mononuclear cells.
There was no significant difference between the two types of PRP preparations and the values were stable in time (p > 0.05).

*8. Recovery of white blood cells subsets in PRP preparations*

See Figure 10.

Table 17

|  | RegenBCT PRP | RegenBCT-HA PRP | p values |
|---|---|---|---|
| Mononuclear cells recovery | 29.77 $\pm$ 9.17 % | 37.87 $\pm$ 25.17 % | ns p>0.05 |
| Granulocytes recovery | 4.62 $\pm$ 2.45 % | 5.48 $\pm$ 4.92 % | ns p>0.05 |
| Mean values obtained from 23 donors | | | |

The preferential removal of granulocytes resulted in a granulocyte recovery of around 5% while around 30% of the mononuclear cells were recovered. There was no significant difference between the two sorts PRP preparations (p > 0.05). The values were stable in time (data not shown).

## 9. Concentration of platelets (PLT) in whole blood and in PRP preparations

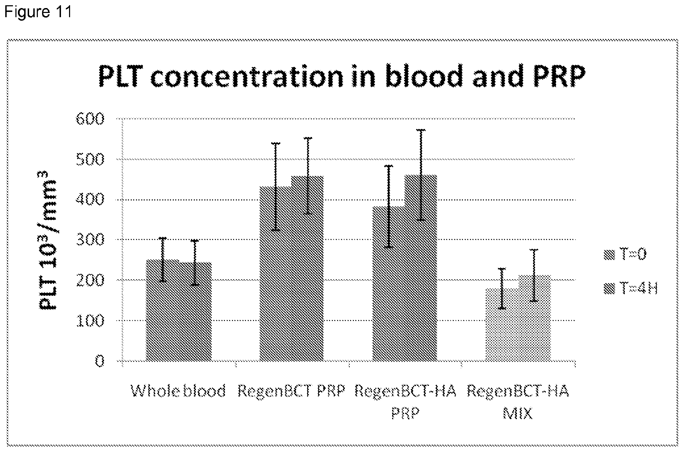

See Figure 11.

Table 18

|  | PLT $10^3$/mm$^3$ at T=0 | PLT $10^3$/mm$^3$ at T=4H | p value T=0 vs T=4H |
|---|---|---|---|
| Whole blood | 251.43 ± 53.87 | 244.30 ± 54.40 | ns p>0.05 |
| RegenBCT PRP | 431.87 ± 106.87 | 459.26 ± 93.64 | ns p>0.05 |
| RegenBCT-HA PRP | 381.91 ± 100.15 | 460.52 ± 111.75 | ***p<0.001 |
| RegenBCT-HA MIX | 179.24 ± 48.93 | 213.12 ± 63.47 | ns p>0.05 |
| p value WB vs PRP | ***p<0.001 | ***p<0.001 | |
| p value WB vs PRP-HA | ***p<0.001 | ***p<0.001 | |
| p value PRP vs PRP-HA | *p<0.05 | ns p>0.05 | |
| Mean values obtained from 23 donors; (WB: whole blood, ns: non-significant; *: significant, ***: highly significant) | | | |

Both type of tubes allowed an augmentation of the platelet concentration in PRP when compared to whole blood (p < 0.001, for both tubes).
At time t=0, the platelet concentration was higher in RegenBCT PRP than in PRP from RegenBCT-HA tubes (p < 0.05), however these values were equivalent after four hours (p > 0.05). The increase in platelet concentration at time T=4H, observed in the PRP obtained from the RegenBCT-HA tubes, is probably due to an underestimation of this value at time T=0, as the platelet concentration was stable in time for both blood and RegenBCT PRP.
The estimated final platelet concentration in the PRP-HA mixes was 179.10$^3$/mm$^3$ at T=0 and 213.10$^3$/mm$^3$ after four hours.

## 10. Platelet concentration factor in PRP preparations

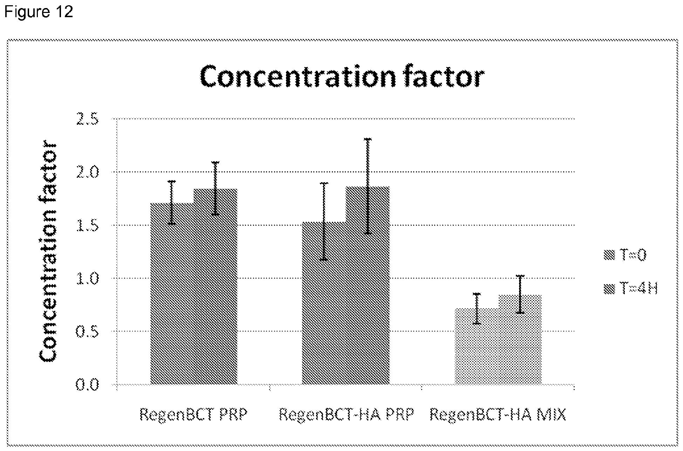

See Figure 12.

Table 19

|  | PLT concentration factor at T=0 | PLT concentration factor at T=4H | p value T=0 vs T=4H |
|---|---|---|---|
| RegenBCT PRP | 1.71 ± 0.20 | 1.85 ± 0.24 | ns p>0.05 |
| RegenBCT-HA PRP | 1.53 ± 0.36 | 1.86 ± 0.45 | ***p<0.001 |
| RegenBCT-HA MIX | 0.71 ± 0.14 | 0.85 ± 0.17 | *p<0. 05 |
| p value PRP vs PRP-HA | ns p>0.05 | ns p>0.05 | |
| Mean values obtained from 23 donors; (ns: non-significant, *: significant, ***: highly significant) | | | |

The platelet concentration factor was equivalent for both type of PRP at T=0 and four hours later (p > 0.05 in both case). While this value was stable in time for RegenBCT PRP (p > 0.05), a significant increase (p < 0.001) of this factor was observed in RegenBCT-HA PRP after four hours. This is in correlation with the observed increase in platelet concentration in these devices at T=4 hours, probably due to an underestimation of the platelet count at T=0.
The estimated final concentration factors in the PRP-HA mixes were 0.71X and 0.85X respectively at time T=0 and four hours later.

## 11. Percentage of platelet recovery in the PRP preparations

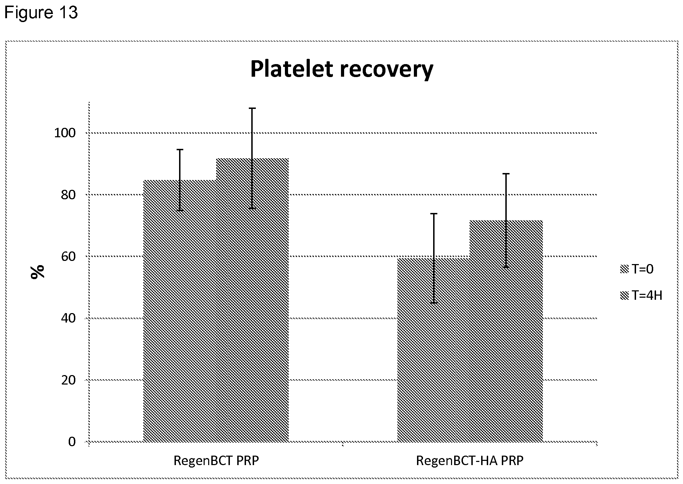

See Figure 13.

Table 20

|  | % PLT recovery at T=0 | % PLT recovery at T=4H | p value T=0 vs T=4H |
|---|---|---|---|
| RegenBCT PRP | 84.80 ± 9.87 | 91.78 ± 16.25 | ns p>0.05 |
| RegenBCT-HA PRP | 59.43 ± 14.48 | 71.70 ± 15.14 | **p<0.01 |
| p value PRP vs PRP-HA | ***p<0.001 | ***p<0.001 |  |
| Mean values obtained from 23 donors; (ns: non-significant; **: very significant, ***: highly significant) | | | |

Platelet recovery with RegenBCT tubes was significantly higher than the platelet recovery obtained with RegenBCT-HA tubes (p < 0.001). RegenBCT PRP results were stable for the four hours of the experiment (p > 0.05), while the observed increase in BCT-HA tubes was significant (p < 0.01). This increase in time is correlated with the increase in platelet concentration observed at T=4 hours in those tube.

The percentage of platelet recovery obtained in RegenBCT-HA tubes is over the FDA requested limit of 50%.

*B. EVALUATION OF PLATELET FUNCTIONALITY*

In the aim to address the platelet functionality and integrity, P-selectin (CD62P) expression in response to ADP, hypotonic stress response, as well as platelet ability to aggregate in response to collagen were evaluated in platelets preparations.

*1. P-selectin (CD 62P) expression on platelets*

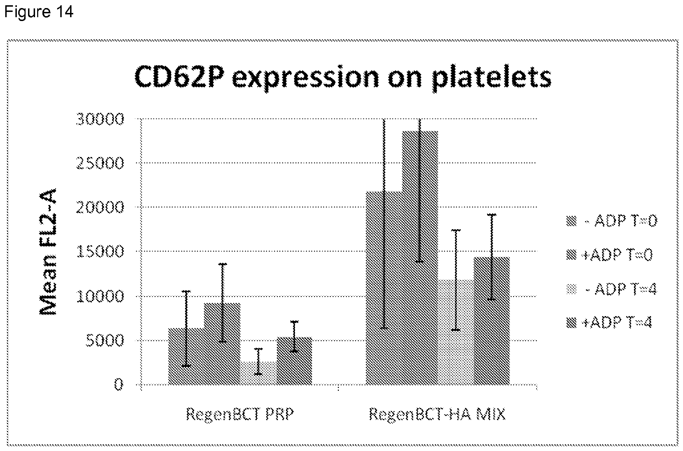

See Figure 14.

Table 21

|  | CD62P at T=0 | | | CD62P at T=4H | | |
|---|---|---|---|---|---|---|
|  | - ADP | + ADP | p value -ADP vs +ADP | - ADP | + ADP | p value -ADP vs +ADP |
| RegenBC T PRP | 6'348.88 ± 4'202.51 | 9'271.71 + 4'381.84 | ** p<0.01 | 2'569.94± 1'417.00 | 5'408.44± 1'639.89 | ** p<0.01 |
| RegenBC T-HA MIX | 21'872.13 ± 15'467.92 | 28'680.78 ± 14'760.51 | * p<0.05 | 11'837.87 ± 5'627.73 | 14'422.91 ± 4'805.49 | ns p>0.05 |
| Mean values obtained from 23 donors; (ns: non-significant; **: very significant, ***: highly significant) | | | | | | |

The mean value of fluorescence for CD62P (P-selectin) was measured on gated CD61 positive platelets in the RegenBCT PRP and in the RegenBCT-HA mix, either without stimulation (-ADP) or with ADP stimulation (+ADP), at time T=0 and 4 hours after blood collection.

We were able to stain platelets in the PRP-HA mixes however in those preparations we observed a high background for both types of fluorescence (in FL1 for CD61-FITC and in FL2 for CD62P-PE) probably due to the presence of hyaluronic acid. Thus the values obtained for the two types of devices could not be directly compared. Moreover a high variation between individuals was observed, resulting in high standard deviation values.

Nevertheless the same pattern of answer to specific ADP stimulation was observed in the two types of platelet preparations. Platelets on resting state at time 0 (-ADP T=0, light blue columns in the graph) expressed a basal level of P selectin. This basal level was significantly lower (p < 0.001, not shown in the table) after four hours (-ADP T=4H, pink columns in the graph).

After specific activation with ADP at time T=0 (+ADP T=0, dark blue columns in the graph), a significant (p < 0.01) increased of the P-selectin expression was observed for the two kinds of platelet suspensions when compared to resting state controls (-ADP T=0).

An increase in fluorescence for CD62P in response to ADP was also observed at T=4H (+ADP T=4H, red columns in the graph). However, this response was significant (p < 0.05) only for the platelets in RegenBCT PRP.

*2. Hypotonic stress response of the platelets*

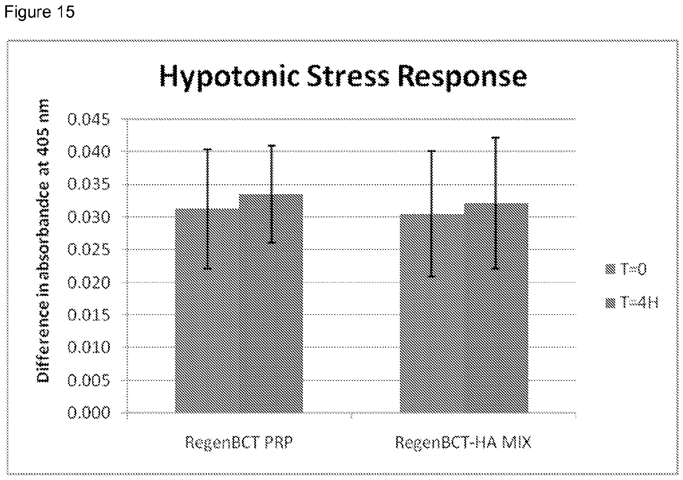

See Figure 15.

Table 22

| | HSR at T=0 | HSR at T=4H | p value T=0 vs T=4H |
|---|---|---|---|
| RegenBCT PRP | $0.031 \pm 0.009$ | $0.034 \pm 0.007$ | ns p>0.05 |
| RegenBCT-HA MIX | $0.030 \pm 0.010$ | $0.032 \pm 0.010$ | ns p>0.05 |
| P value PRP vs PRP HA MIX | ns p>0.05 | ns p>0.05 | |
| Mean values obtained from 23 donors; (ns: non-significant) | | | |

The above table and associated graph represent the mean $\pm$ SD absorbance variation in light absorbance at 405 nm observed during an hypotonic stress induced by adding distilled water on platelet suspensions isolated with the two devices.

The difference between the absorbance readings at 5 min (maximum absorbance) and at 30 sec (minimum absorbance) was defined as the hypotonic stress response (HSR) of the platelet preparation.

At time T=0 platelets in the RegenBCT PRP and in the RegenBCT-HA mix were able to respond to the hypotonic stress with the same amplitude (p > 0.05). This ability to resist to a hypotonic stress was maintained at the same level (p > 0.05) during the four hours of the experiment.

*3. Platelet aggregation*

Figure 16

Table 23

| | Aggregation at T=0 | Aggregation at T=4H | p value T=0 vs T=4H |
|---|---|---|---|
| RegenBCT PRP | $65.08 \pm 15.82$ | $58.50 \pm 17.14$ | ns p>0.05 |
| RegenBCT-HA PRP | $23.60 \pm 8.39$ | $25.30 \pm 9.88$ | ns p>0.05 |
| p value PRP vs PRP-HA | ***p<0.001 | ***p<0.001 | |
| Mean values obtained from 20 donors; (ns: non-significant; ***: highly significant) | | | |

The platelet aggregation could not be measured by turbidimetry in PRP-HA mix, due to the viscosity of the product. Therefore we could only test the aggregation on the PRP fractions obtained after removal of the hyaluronic acid in the RegenBCT-HA tubes. Like for the hematological counts, platelet aggregation at time T=0 was quantified on PRP in RegenBCT-HA tubes from group B, while results at time T=4H were measured in PRP obtained from RegenBCT-HA tubes from group C.

In addition, due to a technical problem we could not give results for three donors.

Both platelets preparations were able to aggregate in response to collagen activation. However the level of response was lower for the platelets prepared with RegenBCT-HA tubes (p < 0.001). The level of response remained unchanged (p > 0.05) when measured after four hours in both PRP preparations.

Discussion

Hyaluronic acid is a major component of the extracellular matrix. It has not only a structural role, forming a three-dimensional scaffold, but also contributes to cell to substrate adhesion, cell to cell contact, cell migration and proliferation. The RegenBCT-HA tube is a new device that allows the preparation, in a single step procedure, of platelet rich plasma directly combined with a hyaluronic acid solution.

In this study, we evaluated the performances of the RegenBCT-HA tubes regarding blood components separation, compared to the performance of the RegenBCT tube, device for the standard preparation of PRP. This was assessed using the parameters recommended by the US Food and Drug Administration (US FDA) which are volumes of the platelet concentrate, platelet and cell counts, and pH of the products. Moreover the viability and functionality of the platelets obtained were also investigated.

The blood of 23 healthy donors was treated in parallel with the two devices and the PRP obtained were analyzed at time 0 and 4 hours after blood collection.

The RegenBCT tube is designed for the preparation of 4 to 5 ml of PRP from 8 to 9 ml of blood. In the RegenBCT-HA tube the vacuum and the anticoagulant volume have been adapted for the collection 4 to 5 ml of blood, in order to obtain 2 to 3 ml of anticoagulated plasma that will be combined with the 2 ml of hyaluronic acid solution contained in the device. The final volume of the PRP-HA mix is around 5 ml, with an approximate ratio of PRP to hyaluronic acid solution of 50%.

In both devices the blood component separation is done by a single step centrifugation. In RegenBCT-HA tubes the hyaluronic acid floats over the plasma at the end of the centrifugation and must be mechanically mixed with the PRP to obtain the final product. As the cell counting could not be done on the PRP-HA mix, due to the viscosity of the final product, we evaluated the cell and platelet concentrations only in the PRP part, by removing the floating hyaluronic acid. Consequently we had to withdraw blood in three RegenBCT-HA tubes, for each donor, in order to have a PRP-HA mix and two PRPs for the measure at time 0 and at time T=4 hours. For the evaluation at T=4H, the RegenBCT-HA tubes were left untouched in vertical position after the centrifugation, and the floating hyaluronic acid was removed only after four hours just before the blood cell counting.

The resulting PRP volumes as well as the amount of hyaluronic acid removed were quantified. There was no statistical difference between the volume obtained at T=0 and four hours later, thus allowing us to compare the data obtained at the two time points.

The pH of the PRP-HA mix was like the pH of the RegenBCT PRP over the FDA requested standard limit of 6.2 for platelet preparation. The pHs of PRP and of PRP-HA MIX were slightly higher than the blood pH, the highest values were observed in the PRP. Although minimal, the differences in pH between the different products were significant. A small increase of the pH value of the two platelet preparations was observed during the 4 hours of the experiment, contrary to the pH of the whole blood which did not vary however this increase was not clinically significant.

Concerning blood cell separation, both systems showed equivalent results in removing red blood cells with a recovery inferior to 0.5%. An equivalent reduction of the white blood cells content in PRP, compared to whole blood, was obtained in the two types of devices. Around 85% of the leukocytes were removed. Furthermore, the same repartition in white blood cells subsets was found in the two kind of PRP, demonstrating the equivalency of the two devices for the preparation of PRP with a low contamination of granulocytes, known to be proinflammatory.

Although non-significant the concentrations in red and white blood cells in the PRP obtained with the RegenBCT-HA tubes were slightly higher than in RegenBCT PRP. This is due to the fact that some plasma was removed with the hyaluronic acid, thus reducing the volume in which the cells were resuspended. As the cell recovery were equivalent in the two devices, it is unlikely that a significant amount of red and white blood cells were in the removed volume. Therefore we could estimate by calculation the final concentration of those cells in the PRP-HA mix obtained with the RegenBCT-HA tubes.

Both devices allowed the preparation of PRP with a platelet concentration higher than the blood platelet concentration. Unlike the other concentrations that were stable in time, the platelet concentration in PRP from RegenBCT-HA tubes increased in time, and was equivalent to the one obtained in RegenBCT tubes only at T=4H. By correlation the same increase was observed for the platelet concentration factor. As it has been demonstrated, with red and white blood cells, that the presence of hyaluronic acid didn't modify the performance of the thixotropic gel for blood component separation, we can presume that the platelet isolation ability of the gel is also unmodified in RegenBCT-HA tubes. Therefore it is likely that some platelets interacted with the hyaluronic acid during the blood conditioning in this device. Those platelet were probably carried away with the removed hyaluronic acid at time T=0. The increased number of platelets found in the RegenBCT-HA PRP after four hours might therefore be due to the release in the plasma of some platelets from the acid hyaluronic fraction during the length of the experiment. This platelet-hyaluronic acid interaction would also explain the lower values obtained in this device for platelet recovery, around 60 and 70 %, respectively at time T=0 and time T=4H while the recovery in RegenBCT tube was between 85% and 92%. Nevertheless the measured percentages of platelet recovery, although probably underestimated, are well over the FDA requested limit of 50%. The estimated final concentration in the PRP-HA mix is close to the platelet concentration in whole blood, with a concentration factor in the diluted product of around 0.8.

In addition to the performance of the RegenBCT-HA device for blood cell separation, we also tested the functionality and integrity of the platelets obtained.

In vivo the platelets respond to specific ADP stimulation by increasing the number of P-selectin (CD62P) molecules expressed on their outer membrane. We tested this ability on the platelets obtained in the PRP-HA mix. The high fluorescence backgrounds, seen in the samples containing hyaluronic acid, prevented us for a direct comparison with the results obtained in RegenBCT PRP. Moreover a high variation between individuals was observed, resulting in high standard deviation values. Nevertheless we observed similar pattern of answer in the two kind of platelet preparations, with an increase of P selectin expression in response to specific activation with ADP, although non-significant at T=4 for PRP-HA mix.

When distilled water is added in PRP preparations, platelets swell and then contract to their normal shape. This ability to resist to a hypotonic stress is dependant of the platelet's biochemical functions integrity and therefore is an indicator of platelet viability. Platelets from both devices were able to resist to such hypotonic stress. The response was of the same amplitude in both types of platelet preparations and remained unchanged during the period of 4 hours

Another functionality of the platelets is their ability to aggregate in response to contact with extracellular matrix molecules, such as collagen. The turbidimetric method used to evaluate this functionality could not be done on platelet samples mixed with hyaluronic acid, due to the viscosity of the product. Therefore, like for hematological counts, we could only test the aggregation on the PRP fractions obtained after removal of the hyaluronic acid in the RegenBCT-HA tubes. Platelets preparations obtained with the two devices were able to aggregate in response to collagen activation and this ability was maintained at the same level at time T=4 hours. However, the aggregation response was reduced for the platelets prepared with RegenBCT-HA tubes. This is consistent with published results on the effect of hyaluronic acid on human platelet aggregation in response to collagen (Aras C., et al., Eur J Ophthalmol. 2006 Mar-Apr;16(2):306-10; Bracey AW., et al., Am J Hematol. 1987 Mar;24(3):247-57).

Taken together, these last three experiments allowed us to demonstrate that the platelets obtained with the RegenBCT-HA tube, although biased by the presence of hyaluronic acid, had maintained their integrity and functionality.

Conclusions

The RegenBCT-HA tube is an innovative medical device that allows the preparation of PRP combined to a hyaluronic acid solution, by a single step procedure in a closed circuit system. The PRP obtained with this device has a high platelet recovery (60-70%), a partial recovery of white blood cells (around 15%), with a selective depletion (95%) of granulocytes and almost no red blood cell contamination (<0.5%). These results are comparable to the results obtained with another device for PRP isolation, the RegenBCT tube, unsurprisingly as the two devices use the same gel technology for cell separation. The only discrepancy was observed for platelet recovery which was higher in RegenBCT PRP, however as we could not evaluate the quantity of platelets bound to hyaluronic acid, this parameter was probably under estimated. Nevertheless the measured value is well over the over the FDA requested limit of 50% for platelet recovery in PRP device. The PRP obtained is mixed with the hyaluronic acid solution, contained in the device, at a ratio of around 50%. The resulting diluted product is a platelet suspension in a three dimensional scaffold with a concentration near the physiological blood value, in which the platelets have maintained their integrity and functionality.

**Example 4**

Efficacy of the HA-PRP formulations has been assessed in patients using the REGENBCT and REGENBCT-HA tubes as described in Example 3. As shown in Figure 19, evidence of HA and growth factors (GF) affinity is demonstrated for PDGF and EGF. A much stronger GF release is observed in a combination of HA with PRP than PRP alone. Release in pg/ml of PDGF-AA, PDGF-BB and EGF at day 0 (T0) for a PRP preparation alone (BCT T0) versus a combination of PRP and HA (BCT+IALUR T0) is shown in Figure 19.

Surprisingly, the early phase GF delivery is distinct between a PRP preparation alone (BCT) versus a combination of PRP and HA (BCT HA), see Figure 20. Release in pg/ml of PDGF-BB in two cases at day 0 (T0), day 3 (T3) and day 5 (T5) for a PRP preparation alone (BCT) versus a combination of PRP and HA (BCT HA) is shown in Figure 20.

Release in pg/ml of EGF in three different patients (patients 1, 2 or 3) at day 0 (T0), day 3 (T3), day 5 (T5), day 7(T7) and day 10 (T10) for a PRP preparation alone (BCT), is shown in Figure 21-A versus a combination of PRP and HA (BCT HA) in Figure 21-B. The median release in pg/ml of EGF at day 0 (T0), day 3 (T3), day 5 (T5), day 7(T7) and day 10 (T10) for a PRP preparation alone (BCT) versus a combination of PRP and HA (BCT HA) is shown in Figure 21-C.

A combination of PRP and HA is very effective in the early phase with much higher GF release, particularly for PDGF-BB until about day 3 or day 4 and for EGF until about day 1 or day 2. This is particularly important in all instances where a rapid and strong release of GF is required, for example in wound care. Such early phase GF release enhances wound healing time and rapidly decreases pain. For example, a HA-PRP formulation according to the disclosure is useful in acute pain.

**Claims**

1. A composition comprising at least one low molecular weight hyaluronic acid and at least one high molecular weight hyaluronic acid **characterized in that**:

   - said low molecular weight hyaluronic acid is 600kDa or less than 600 KDa, and
   - said high molecular weight hyaluronic acid is 4000 KDa or above 4000 KDa,

wherein:

- the ratio of low molecular weight hyaluronic acid to high molecular weight hyaluronic acid is 2:3 and wherein the total concentration is 2.2% to 2.8%, and further comprising:

i) an anticoagulant and/or a thixotropic gel.

2. The composition of claim 1, wherein the anticoagulant is citrate or sodium citrate.

3. The composition according to claim 1 or claim 2, further comprising:

i) a coagulation activator, thrombin serum, tricalcium phosphate (TCP), a bone substitute, calcium gluconate, calcium saccharate, chitosan, fibroins, silk-fibroin proteins, growth factors, mannitol, collagen, albumin, ascorbic acid, cream, fat cells, fat tissue, bone marrow concentrate, lubricin, cd-gelatin, botulinum toxin and/or one or more cell extracts which are not embryonic stem cell extracts, and/or
ii) analgesic compounds, antibacterial compounds, including bactericidal and bacteriostatic compounds, antibiotics such as adriamycin, erythromycin, gentimycin, penicillin, tobramycin, antifungal compounds, anti-inflammatories, antiparasitic compounds, antiviral compounds, enzymes, enzyme inhibitors, glycoproteins, growth factors, lymphokines, cytokines, hormones, steroids, glucocorticosteroids, immunomodulators, immunoglobulins, minerals, neuroleptics, proteins, peptides, lipoproteins, tumoricidal compounds, tumorstatic compounds, toxins and vitamins such as Vitamin A, Vitamin E, Vitamin B, Vitamin C, Vitamin D.

4. The composition according to any of claims 1-3, wherein said composition is steam sterilized.

**Patentansprüche**

1. Zusammensetzung umfassend zumindest eine Hyaluronsäure mit niedrigem Molekulargewicht und mindestens eine Hyaluronsäure mit hohem Molekulargewicht, **dadurch gekennzeichnet, dass**:

- die Hyaluronsäure mit niedrigem Molekulargewicht 600 kDa oder weniger als 600 kDa beträgt, und
- die Hyaluronsäure mit hohem Molekulargewicht 4000 kDa oder mehr als 4000 kDa beträgt,
wobei das Verhältnis der Hyaluronsäure mit niedrigem Molekulargewicht zu der Hyaluronsäure mit hohem Molekulargewicht 2:3 beträgt und wobei die Gesamtkonzentration 2,2% bis 2,8% beträgt und ferner umfassend:

i) ein Antikoagulans und/oder ein thixotropes Gel.

2. Zusammensetzung nach Anspruch 1, bei der das Antikoagulans Citrat oder Natriumcitrat ist.

3. Zusammensetzung nach Anspruch 1 oder Anspruch 2, ferner umfassend:

i) einen Gerinnungsaktivator, Thrombinserum, Tricalciumphosphat (TCP), einen Knochenersatz, Calciumgluconat, Calciumsaccharat, Chitosan, Fibroine, Fibroin-Seidenproteine, Wachstumsfaktoren, Mannit, Kollagen, Albumin, Ascorbinsäure, Creme, Fettzellen, Fettgewebe, Knochenmarkkonzentrat, Lubricin, CD-Gelatine, Botulinumtoxin und/oder eines oder mehrere Zellextrakte, welches nicht embryonale Stammzellenextrakte sind, und/oder
ii) analgetische Verbindungen, antibakterielle Verbindungen, umfassend bakterizide und bakteriostatische Verbindungen, Antibiotika, wie beispielsweise Adriamycin, Erythromycin, Gentimycin, Penicillin, Tobramycin, antimykotische Verbindungen, entzündungshemmende Mittel, antiparasitische Verbindungen, antivirale Verbindungen, Enzyme, Enzyminhibitoren, Glykoproteine, Wachstumsfaktoren,
Lymphokine, Zytokine, Hormone, Steroide, Glukokortikosteroide, Immunmodulatoren, Immunglobuline, Mineralien, Neuroleptika, Proteine, Peptide, Lipoproteine, tumorizide Verbindungen, tumorstatische Verbindungen, Toxine und Vitamine, wie beispielsweise Vitamin A, Vitamin E, Vitamin B, Vitamin C, Vitamin D.

4. Zusammensetzung nach einem der Ansprüche 1-3, bei der die Zusammensetzung dampfsterilisiert ist.

**Revendications**

1. Composition comprenant au moins un acide hyaluronique de bas poids moléculaire et au moins un acide hyaluronique de haut poids moléculaire **caractérisée en ce que** :

   - ledit acide hyaluronique de bas poids moléculaire est de 600 kDa ou moins que 600 kDa, et
   - ledit acide hyaluronique de haut poids moléculaire est de 4000 kDa ou au-dessus de 4000 kDa,

   dans laquelle :

   - le rapport de l'acide hyaluronique de bas poids moléculaire à l'acide hyaluronique de haut poids moléculaire est de 2 : 3 et où la concentration totale est de 2,2 % à 2,8 %, et comprenant en outre :

      i) un anticoagulant ou un gel thixotropique.

2. Composition selon la revendication 1, dans laquelle l'anticoagulant est le citrate ou le citrate de sodium.

3. Composition selon la revendication 1 ou la revendication 2, comprenant en outre :

      i) un activateur de coagulation, du sérum avec thrombine, du phosphate tricalcique (TCP), un substitut d'os, du gluconate de calcium, du saccharate de calcium, du chitosan, des fibroïnes, des protéines de fibroïne de la soie, des facteurs de croissance, du mannitol, du collagène, de l'albumine, de l'acide ascorbique, une crème, des cellules grasses, du tissu gras, un concentré de moelle osseuse, de la lubricine, de la cd-gélatine, la toxine du botulinum et/ou un ou plusieurs extraits cellulaires qui ne sont pas des extraits de cellules souches embryonnaires, et/ou
      ii) des composés analgésiques, des composés antibactériens, y compris des composés bactéricides et bactériostatiques, des antibiotiques tels que l'adriamycine, l'érythromicine, la gentimycine, la pénicilline, la tobramycine, des composés antifongiques, des anti-inflammatoires, des composés antiparasitaires, des composés antiviraux, des enzymes, des inhibiteurs d'enzyme, des glycoprotéines, des facteurs de croissance, des lymphokines, des cytokines, des hormones, des stéroïdes, des glucocorticostéroïdes, des immunomodulateurs, des immunoglobulines, des minéraux, des neuroleptiques, des protéines, des peptides, des lipoprotéines, des composés tumoricidaux, des composés statiques de tumeur, des toxines et des vitamines telles que la vitamine A, la vitamine E, la vitamine B, la vitamine C, la vitamine D.

4. Composition selon l'une quelconque des revendications 1 à 3, dans laquelle ladite composition est stérilisée à la vapeur.

Figure 1

Figure 2

Figure 3

1=Unterbahnfolie
   bottom web film
2=Vorwärm-Formstation
   preheating and forming station
3=Siegelstation
   sealing station
4=Kamerasystem
   camerasystem
5=Oberbahnfolie
   top web film
6=Drucker -printer
7=Bedientableau
   operating board
8=Komplettschnittstanze
   die cut station
9=Restgitteraufrollung
   scrap web winder
10=Auslaufförderband
   discharge conveyor belt
11=Transfereinheit
   picker

EP 2 771 241 B1

Figure 4

Volume of blood withrawn and of PRP or PRP-HA MIX obtained

Figure 5

**Mean blood and PRP pH**

Figure 6

Figure 7

WBC concentration in blood and PRP

Figure 8

Figure 9

Figure 10

WBC subsets recovery at T=0

Figure 11

Figure 12

Concentration factor

Figure 13

Figure 14

Figure 15

**Hypotonic Stress Response**

Figure 16

Figure 17

Viscosité=Viscosity

Figure 18

Viscosity of HAs plotted against concentration (w/v)

Figure 19

Figure 20

PDGF-BB CASE 1

PDGF-BB CASE 2

Figure 21

**EP 2 771 241 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011110948 A
- US 6472162 B
- WO 201110948 A

### Non-patent literature cited in the description

- **MARX et al.** *J. Oral Maxillofac. Surg.,* 2004, vol. 62, 489-496
- **FARRUGIA et al.** *J Clin Pathol,* 1989, vol. 42, 1298-1301
- **ARAS C. et al.** *Eur J Ophthalmol.,* March 2006, vol. 16 (2), 306-10
- **BRACEY AW. et al.** *Am J Hematol.,* March 1987, vol. 24 (3), 247-57